(19) Europäisches Patentamt
European Patent Office
Office européen des brevets

(11) Veröffentlichungsnummer: **0 363 582 A1**

(12) **EUROPÄISCHE PATENTANMELDUNG**

(21) Anmeldenummer: **89113967.7**

(22) Anmeldetag: **28.07.89**

(51) Int. Cl.5: **C07D 405/06 , C07D 407/04 , C07D 317/26 , C07D 303/32 , C07C 33/46 , C07C 43/178 , C07C 43/23 , C07C 317/24 , C07C 323/22**

(30) Priorität: **10.08.88 DE 3827134**

(43) Veröffentlichungstag der Anmeldung:
**18.04.90 Patentblatt 90/16**

(84) Benannte Vertragsstaaten:
**BE CH DE ES FR GB IT LI NL SE**

(71) Anmelder: **BAYER AG**
**D-5090 Leverkusen 1 Bayerwerk(DE)**

(72) Erfinder: **Elbe, Hans-Ludwig, Dr.**
**Dasnöckel 59**
**D-5600 Wuppertal 11(DE)**
Erfinder: **Kranz, Eckart, Dr.**
**Am Acker 9**
**D-5600 Wuppertal 1(DE)**
Erfinder: **Brandes, Wilhelm, Dr.**
**Eichendorffstrasse 3**
**D-5653 Leichlingen 1(DE)**
Erfinder: **Dutzmann, Stefan, Dr.**
**Leinenweberweg 33**
**D-4000 Düsseldorf 13(DE)**
Erfinder: **Hänssler, Gerd, Dr.**
**Am Arenzberg 58a**
**D-5090 Leverkusen 3(DE)**

(54) **Substituierte Dioxolane.**

(57) Neue substituierte Dioxolane der allgemeinen Formel (I),

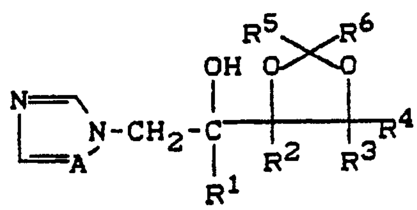

(I)

in der A und $R^1$ bis $R^6$ die in der Beschreibung angegebene Bedeutung haben und deren Verwendung als Mikrobizide. Die neuen substituierten Dioxolane der Formel (I) können hergestellt werden, indem man geeignete Oxiranyldioxolane mit geeigneten Azolen gegebenenfalls in Gegenwart geeigneter Verdünnungsmittel und gegebenenfalls in Gegenwart geeigneter Reaktionshilfsmittel umsetzt und gegebenenfalls anschließend geeignete Säuren oder Metallsalze addiert.

EP 0 363 582 A1

## Substituierte Dioxolane

Die Erfindung betrifft neue substituierte Dioxolane, ein Verfahren zu ihrer Herstellung und ihre Verwendung als Mikrobizide.

Es ist bekannt, daß bestimmte heterocyclisch substituierte Carbinole fungizide Eigenschaften besitzen (vgl. EP-OS 0 055 833 und US-PS 4 417 050). So lassen sich zum Beispiel 4-(4-Chlorphenyl)-3,3-dimethyl-1-(1,2,4-triazol-1-yl)-2-butanol und (4-Chlorphenyl)-(5-pyrimidinyl)-methanol zur Bekämpfung von Pilzen verwenden. Die Wirksamkeit dieser Stoffe ist jedoch insbesondere bei niedrigen Aufwandmengen nicht immer völlig zufriedenstellend.

Es wurden nun neue substituierte Dioxolane der Formel

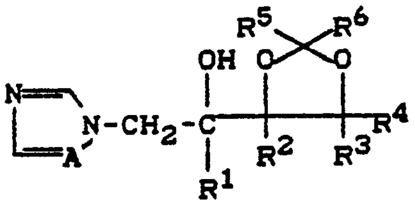

(I),

in welcher

$R^1$ für gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkylalkyl, gegebenenfalls substituiertes Aryl, gegebenenfalls substituiertes Arylalkyl, gegebenenfalls substituiertes Aryloxyalkyl, gegebenenfalls substituiertes Arylthioalkyl, gegebenenfalls substituiertes Arylsulfinylalkyl, gegebenenfalls substituiertes Arylsulfonylalkyl, gegebenenfalls substituiertes Aralkyloxyalkyl, gegebenenfalls substituiertes Aralkylthioalkyl, gegebenenfalls substituiertes Aralkylsulfinylalkyl oder gegebenenfalls substituiertes Aralkylsulfonylalkyl steht,

$R^2$, $R^3$, $R^4$, $R^5$ und $R^6$ unabhängig voneinander für Wasserstoff, Alkyl oder Cycloalkyl stehen oder

$R^3$ und $R^4$ gemeinsam für zweifach verknüpftes Alkandiyl stehen und/oder

$R^5$ und $R^6$ gemeinsam für zweifach verknüpftes Alkandiyl stehen und

A für Stickstoff oder eine CH-Gruppe steht,

sowie deren Säureadditions-Salze und Metallsalz-Komplexe gefunden.

Die erfindungsgemäßen Stoffe der Formel (I) besitzen in Abhängigkeit von der Art der Substituenten $R^1$ bis $R^6$ zwei oder mehr asymmetrisch substituierte Kohlenstoffatome und können daher in Form von optischen Isomeren oder Diastereomeren auftreten. Die Erfindung betrifft sowohl die reinen Isomeren als auch deren Gemische in unterschiedlicher Zusammensetzung.

Weiterhin wurde gefunden, daß man substituiertes Dioxolane der Formel (I) sowie deren Säureadditions-Salze und Metallsalz-Komplexe erhält, wenn man Oxiranyl-dioxolane der Formel

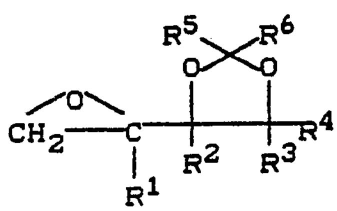

(II),

in welcher

$R^1$, $R^2$, $R^3$, $R^4$, $R^5$ und $R^6$ die oben angegebene Bedeutung haben,

mit Azolen der Formel

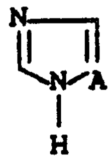

(III),

in welcher
A die oben angegebene Bedeutung hat,
gegebenenfalls in Gegenwart eines Verdünnungsmittels und gegebenenfalls in Gegenwart eines Reaktionshilfsmittels umsetzt und gegebenenfalls anschließend eine Säure oder ein Metallsalz addiert.

Schließlich wurde gefunden, daß die neuen substituierten Dioxolane der Formel (I) sowie deren Säureadditionssalze und Metallsalzkomplexe sehr gute fungizide Eigenschaften besitzen.

Überraschenderweise zeigen die erfindungsgemäßen substituierten Dioxolane der Formel (I) eine erheblich bessere fungizide Wirksamkeit als 4-(4-Chlorphenyl)-3,3-dimethyl-1-(1,2,4-triazol-1-yl)-2-butanol und (4-Chlorphenyl)-(5-pyrimidinyl)-methanol, welches konstitutionell und wirkungsmäßig ähnliche, vorbekannte Stoffe sind.

Die erfindungsgemäßen substituierten Dioxolane sind durch die Formel (I) allgemein definiert.

Alkyl ist im folgenden, falls nicht anders definiert, in einzelnen und in Alkyl enthaltenden Resten zum Beispiel geradkettiges oder verzweigtes Alkyl mit 1 bis 12, vorzugsweise 1 bis 8, insbesondere 1 bis 7 und vor allem 3 bis 7 Kohlenstoffatomen.

Alkenyl und Alkinyl sind im folgenden, falls nicht anders definiert, zum Beispiel geradkettiges oder verzweigtes Alkenyl bzw. Alkinyl mit 2 bis 12, vorzugsweise 2 bis 8, insbesondere 2 bis 7 und vor allem 3 bis 7 Kohlenstoffatomen.

Cycloalkyl enthält im allgemeinen 3 bis 7, vorzugsweise 3 bis 6 Kohlenstoffatome im Cycloalkylteil.

Aryl enthält im allgemeinen 6 bis 10 Kohlenstoffatome im Arylteil, wobei Phenyl-, α-Naphthyl- oder ß-Naphthylreste beispielhaft genannt seien.

Diese Reste können zum Beispiel durch ein oder mehrere, gleiche oder verschiedene Substituenten der folgenden Gruppen substituiert sein:

Halogenatome wie Fluor, Chlor, Brom und Iod, vorzugsweise Fluor, Chlor und Brom, Cyano, Nitro, jeweils geradkettiges oder verzweigtes Alkyl, Alkoxy, Alkylthio, Alkylsulfinyl oder Alkylsulfonyl mit jeweils 1 bis 8, vorzugsweise 1 bis 6 und insbesondere 1 bis 4 Kohlenstoffatomen im Alkylteil, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, unsubstituiertes oder einfach oder mehrfach, gleich oder verschieden durch Halogen substituiertes, zweifach verknüpftes Dioxyalkylen, Cycloalkyl mit 3 bis 7 Kohlenstoffatomen und unsubstituiertes oder einfach oder mehrfach, gleich oder verschieden durch Halogen und/oder geradkettiges oder verzweigtes Alkyl mit 1 bis 4 Kohlenstoffatomen substituiertes Phenyl oder Phenoxy.

Bevorzugt sind Verbindungen der Formel (I), in denen
$R^1$ für geradkettiges oder verzweigtes Alkyl mit 1 bis 12 Kohlenstoffatomen, geradkettiges oder verzweigtes Alkenyl mit 2 bis 12 Kohlenstoffatomen, geradkettiges oder verzweigtes Alkinyl mit 2 bis 12 Kohlenstoffatomen, Alkoxyalkyl mit 1 bis 8 Kohlenstoffatomen im Alkoxyteil und 1 bis 8 Kohlenstoffatomen im Alkylteil, Alkylthioalkyl mit 1 bis 8 Kohlenstoffatomen im Alkylthioteil und 1 bis 8 Kohlenstoffatomen im Alkylteil, Alkylsulfinylalkyl mit 1 bis 8 Kohlenstoffatomen im Alkylsulfinylteil und 1 bis 8 Kohlenstoffatomen im Alkylteil, Alkylsulfonylalkyl mit 1 bis 8 Kohlenstoffatomen im Alkylsulfonylteil und 1 bis 8 Kohlenstoffatomen im Alkylteil, Alkoximinoalkyl mit 1 bis 8 Kohlenstoffatomen im Alkoxyteil und 1 bis 8 Kohlenstoffatomen im Alkylteil, Hydroximinoalkyl mit 1 bis 8 Kohlenstoffatomen, Cyanalkyl mit 1 bis 8 Kohlenstoffatomen im Alkylteil, Halogenalkyl mit 1 bis 8 Kohlenstoffatomen und 1 bis 9 gleichen oder verschiedenen Halogenatomen, Halogenalkoxyalkyl mit 1 bis 8 Kohlenstoffatomen im Alkoxyteil und 1 bis 8 Kohlenstoffatomen im Alkylteil und mit 1 bis 9 gleichen oder verschiedenen Halogenatomen, Halogenalkylthioalkyl mit 1 bis 8 Kohlenstoffatomen im Alkylthioteil und 1 bis 8 Kohlenstoffatomen im Alkylteil und mit 1 bis 9 gleichen oder verschiedenen Halogenatomen, Halogenalkylsulfinylalkyl mit 1 bis 8 Kohlenstoffatomen im Halogenalkylteil und 1 bis 8 Kohlenstoffatomen im Alkylteil und mit 1 bis 9 gleichen oder verschiedenen Halogenatomen, Halogenalkylsulfonylalkyl mit 1 bis 8 Kohlenstoffatomen im Halogenalkylteil und 1 bis 8 Kohlenstoffatomen im Alkylteil und 1 bis 9 gleichen oder verschiedenen Halogenatomen, für Halogenalkenyl mit 2 bis 12 Kohlenstoffatomen und 1 bis 9 gleichen oder verschiedenen Halogenatomen, Halogenalkinyl mit 2 bis 12 Kohlenstoffatomen und 1 bis 9 gleichen oder verschiedenen Halogenatomen, für Dioxolanylalkyl mit 1 bis 6

Kohlenstoffatomen im Alkylteil, Dithiolanylalkyl mit 1 bis 6 Kohlenstoffatomen im Alkylteil, Dioxanylalkyl mit 1 bis 6 Kohlenstoffatomen im Alkylteil oder für Dithianylalkyl mit 1 bis 6 Kohlenstoffatomen im Alkylteil steht,

oder

$R^1$ für Cycloalkyl mit 3 bis 7 Kohlenstoffatomen steht, wobei jeder dieser Cycloalkylreste einfach oder mehrfach, gleichartig oder verschieden substituiert sein kann durch Halogen und/oder Alkyl mit 1 bis 4 Kohlenstoffatomen,

oder

$R^1$ für Cycloalkylalkyl mit 3 bis 7 Kohlenstoffatomen im Cycloalkylteil und 1 bis 8 Kohlenstoffatomen im Alkylteil steht, wobei jeder dieser Reste im Cycloalkylteil einfach oder mehrfach, gleichartig oder verschieden substituiert sein kann durch Halogen und/oder Alkyl mit 1 bis 4 Kohlenstoffatomen,

oder

$R^1$ für Aryl mit 6 bis 10 Kohlenstoffatomen, Arylalkyl mit 6 bis 10 Kohlenstoffatomen im Arylteil und 1 bis 8 Kohlenstoffatomen im Alkylteil, Aryloxyalkyl mit 6 bis 10 Kohlenstoffatomen im Arylteil und 1 bis 8 Kohlenstoffatomen im Alkylteil, Arylthioalkyl mit 6 bis 10 Kohlenstoffatomen im Arylteil und 1 bis 8 Kohlenstoffatomen im Alkylteil, Arylsulfinylalkyl mit 6 bis 10 Kohlenstoffatomen im Arylteil und 1 bis 8 Kohlenstoffatomen im Alkylteil, Arylsulfonylalkyl mit 6 bis 10 Kohlenstoffatomen im Arylteil und 1 bis 8 Kohlenstoffatomen im Alkylteil, Aralkyloxyalkyl mit 6 bis 10 Kohlenstoffatomen im Arylteil und 1 bis 8 Kohlenstoffatomen in jedem Alkylteil, Aralkylthioalkyl mit 6 bis 10 Kohlenstoffatomen im Arylteil und 1 bis 8 Kohlenstoffatomen in jedem Alkylteil, Aralkylsulfinylalkyl mit 6 bis 10 Kohlenstoffatomen im Arylteil und 1 bis 8 Kohlenstoffatomen in jedem Alkylteil oder für Aralkylsulfonylalkyl mit 6 bis 10 Kohlenstoffatomen im Arylteil und 1 bis 8 Kohlenstoffatomen in jedem Alkylteil steht, wobei jeder der zuvor genannten Aryl-Reste im Arylteil einfach oder mehrfach, gleichartig oder verschieden substituiert sein kann durch Halogen,Cyano, Nitro, geradkettiges oder verzweigtes Alkyl, geradkettiges oder verzweigtes Alkoxy, geradkettiges oder verzweigtes Alkylthio, geradkettiges oder verzweigtes Alkylsulfinyl oder geradkettiges oder verzweigtes Alkylsulfonyl mit jeweils 1 bis 4 Kohlenstoffatomen, geradkettiges oder verzweigtes Halogenalkyl, geradkettiges oder verzweigtes Halogenalkoxy, geradkettiges oder verzweigtes Halogenalkylthio, geradkettiges oder verzweigtes Halogenalkylsulfinyl oder geradkettiges oder verzweigtes Halogenalkylsulfonyl mit jeweils 1 bis 4 Kohlenstoffatomen und 1 bis 9 gleichen oder verschiedenen Halogenatomen, geradkettiges oder verzweigtes Alkoxycarbonyl oder geradkettiges oder verzweigtes Alkoximinoalkyl mit jeweils 1 bis 4 Kohlenstoffatomen in den einzelnen Alkylteilen, unsubstituiertes oder einfach oder mehrfach, gleich oder verschieden durch Halogen substituiertes, zweifach verknüpftes Dioxyalkylen, Cycloalkyl mit 3 bis 7 Kohlenstoffatomen und unsubstituiertes oder einfach oder mehrfach, gleich oder verschieden durch Halogen und/oder geradkettiges oder verzweigtes Alkyl mit 1 bis 4 Kohlenstoffatomen substituiertes Phenyl oder Phenoxy,

$R^2$, $R^3$, $R^4$, $R^5$ und $R^6$ unabhängig voneinander jeweils für Wasserstoff, für geradkettiges oder verzweigtes Alkyl mit 1 bis 6 Kohlenstoffatomen oder für Cycloalkyl mit 3 bis 7 Kohlenstoffatomen stehen, wobei auch entweder $R^3$ und $R^4$ gemeinsam oder $R^5$ und $R^6$ gemeinsam für einen jeweils zweifach verknüpften Alkandiylrest mit 2 bis 6 Kohlenstoffatomen stehen können und

A für Stickstoff oder eine CH-Gruppe steht.

Besonders bevorzugt sind Verbindungen der Formel (I), in denen

$R^1$ für jeweils geradkettiges oder verzweigtes Alkyl mit 1 bis 8 Kohlenstoffatomen, Alkenyl mit 2 bis 8 Kohlenstoffatomen oder Alkinyl mit 2 bis 8 Kohlenstoffatomen, für geradkettiges oder verzweigtes Alkoxyalkyl, geradkettiges oder verzweigtes Alkylthioalkyl, geradkettiges oder verzweigtes Alkylsulfinylalkyl, geradkettiges oder verzweigtes Alkylsulfonylalkyl, geradkettiges oder verzweigtes Alkoximinoalkyl, geradkettiges oder verzweigtes Hydroximinoalkyl oder geradkettiges oder verzweigtes Cyanalkyl mit jeweils 1 bis 6 Kohlenstoffatomen in den einzelnen Alkylteilen, für geradkettiges oder verzweigtes Halogenalkyl, geradkettiges oder verzweigtes Halogenalkoxyalkyl, geradkettiges oder verzweigtes Halogenalkylthioalkyl, geradkettiges oder verzeigtes Halogenalkylsulfinylalkyl oder geradkettiges oder verzweigtes Halogenalkylsulfonylalkyl mit jeweils 1 bis 4 Kohlenstoffatomen in den einzelnen Alkylteilen und jeweils 1 bis 5 gleichen oder verschiedenen Halogenatomen, für geradkettiges oder verzweigtes Halogenalkenyl oder geradkettiges oder verzweigtes Halogenalkinyl mit jeweils 3 bis 8 Kohlenstoffatomen und jeweils 1 bis 5 gleichen oder verschiedenen Halogenatomen, für Dioxolanylalkyl, Dithiolanylalkyl, Dioxanylalkyl oder Dithianylalkyl mit jeweils 1 bis 4 Kohlen stoffatomen im geradkettigen oder verzweigten Alkylteil oder für jeweils unsubstituiertes oder im Cycloalkylteil ein- bis fünffach, gleich oder verschieden substituiertes Cycloalkylalkyl oder Cycloalkyl mit jeweils 3 bis 6 Kohlenstoffatomen im Cycloalkylteil und 1 bis 6 Kohlenstoffatomen im geradkettigen oder verzweigten Alkylteil steht, wobei als Substituenten im Cycloalkylteil jeweils in Frage kommen: Fluor, Chlor oder Brom oder geradkettiges oder verzweigtes Alkyl mit 1 bis 4 Kohlenstoffatomen;

oder für jeweils unsubstituiertes oder im Arylteil ein- bis fünffach, gleich oder verschieden substituiertes

Arylalkyl, Aryloxyalkyl, Arylthioalkyl, Arylsulfinylalkyl, Arylsulfonylalkyl, Aralkyloxyalkyl, Aralkylthioalkyl, Aralkylsulfinylalkyl, Aralkylsulfonylalkyl, Aryl, mit jeweils 1 bis 6 Kohlenstoffatomen in den einzelnen geradkettigen oder verzweigten Alkylteilen steht, wobei Aryl jeweils für Phenyl, α-Naphthyl oder ß-Naphthyl steht, wobei als Phenylsubstituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Propan-1,3-diyl, Butan-1,4-diyl, Pentan-1,5-diyl, Dimethylpropan-1,3-diyl, Tetramethylpropan-1,3- diyl, Dimethylbutan-1,4-diyl, Tetramethylbutan- 1,4-diyl, Cyclopropyl, Cyclobutyl, Cyclopentyl, Cylohexyl, Trifluormethyl, Dichlorfluormethyl,Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen oder jeweils unsubstituiertes oder ein- bis dreifach, gleich oder verschieden durch Methyl, Fluor oder Chlor substituiertes Phenyl oder Phenoxy;
und wobei als Naphthylsubstituenten jeweils in Frage kommen: Fluor, Chlor, Brom, Cyano, Methyl, Ethyl, n- oder i-Propyl,
$R^2$, $R^3$, $R^4$, $R^5$ und $R^6$ unabhängig voneinander jeweils für Wasserstoff, Methyl, Ethyl, n- oder i-Propyl, n-, i-, s- oder t-Butyl, für Cyclopentyl oder für Cyclohexyl stehen, wobei auch entweder $R^3$ und $R^4$ gemeinsam oder $R^5$ und $R^6$ gemeinsam für einen jeweils zweifach verknüpften Butan-1,4-diylrest oder Pentan-1,5-diylrest stehen können und
A für Stickstoff oder eine CH-Gruppe steht.

Ganz besonders bevorzugt sind Verbindungen der Formel (I), in denen
$R^1$ für geradkettiges oder verzweigtes Alkyl mit 1 bis 7 Kohlenstoffatomen, geradkettiges oder verzweigtes Alkenyl mit 2 bis 7 Kohlenstoffatomen, geradkettiges oder verzweigtes Alkinyl mit 2 bis 7 Kohlenstoffatomen, für geradkettiges oder verzweigtes Methoxyalkyl, geradkettiges oder verzweigtes Dimethoxyalkyl, geradkettiges oder verzweigtes Trimethoxyalkyl, geradkettiges oder verzweigtes Methylthioalkyl, geradkettiges oder verzweigtes Dimethylthioalkyl, geradkettiges oder verzweigtes Trimethylthioalkyl, geradkettiges oder verzweigtes Methylsulfinylalkyl, geradkettiges oder verzweigtes Methylsulfonylalkyl, geradkettiges oder verzweigtes Methoximinoalkyl, geradkettiges oder verzweigtes Hydroximinoalkyl oder geradkettiges oder verzweigtes Cyanalkyl mit jeweils 3 bis 6 Kohlenstoffatomen im Alkylteil, für geradkettiges oder verzweigtes Halogenalkyl, geradkettiges oder verzweigtes Halogenmethoxyalkyl, geradkettiges oder verzweigtes Halogenethoxyalkyl, jeweils geradkettiges oder verzweigtes Halogenmethylthioalkyl oder Halogenmethylsulfinylalkyl oder geradkettiges oder verzweigtes Halogenmethylsulfonylalkyl mit jeweils 1 bis 3 gleichen oder verschiedenen Halogenatomen, insbesondere Fluor, Chlor oder Brom und mit jeweils 3 bis 7 Kohlenstoffatomen im Alkylteil, für geradkettiges oder verzweigtes Halogenalkenyl oder geradkettiges oder verzweigtes Halogenalkinyl mit jeweils 3 bis 7 Kohlenstoffatomen und jeweils 1 bis 3 gleichen oder verschiedenen Halogenatomen, insbesondere Fluor, Chlor oder Brom oder für Dioxolanylalkyl, Dithiolanylalkyl, Dioxanylalkyl oder Dithianylalkyl mit jeweils 1 bis 3 Kohlenstoffatomen im geradkettigen oder verzweigten Alkylteil steht; oder für jeweils unsubstituiertes oder ein- bis fünffach, gleich oder verschieden im Cycloalkylteil durch Fluor, Chlor, Brom oder Methyl substituiertes Cycloalkyl oder Cycloalkylalkyl mit jeweils 3 bis 6 Kohlenstoffatomen im Cycloalkylteil und 1 bis 4 Kohlenstoffatomen im geradkettigen oder verzweigten Alkylteil steht;
oder für jeweils unsubstituiertes oder ein- bis dreifach, gleich oder verschieden substituiertes Phenyl, α-Naphthyl oder ß-Naphthyl oder für einen Rest der Formel

$$Ar-(X)_m-CH_2-;\quad Ar-(X)_m-\overset{\overset{\displaystyle CH_3}{|}}{C}H-\quad \text{oder}\quad Ar-(X)_m-\underset{\underset{\displaystyle CH_3}{|}}{\overset{\overset{\displaystyle CH_3}{|}}{C}}-\quad \text{steht,}$$

wobei
Ar jeweils für unsubstituiertes oder ein- bis dreifach, gleich oder verschieden substituiertes Phenyl, α-Naphthyl oder ß-Naphthyl steht,
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$
oder $-S(O)_n-CH_2-CH_2-$ steht,
m jeweils für eine Zahl 0 oder 1 steht und
n jeweils für eine Zahl 0, 1 oder 2 steht,
wobei als Phenylsubstituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Propan-1,3-diyl, Butan-1,4-diyl, Pentan-1,5-diyl, Dimethylpropan-1,3-diyl, Tetramethylpropan-1,3-diyl, Dimethylbutan-1,4-diyl, Tetramethylbutan-1,4-diyl, Cyclopropyl, Cyclobutyl, Cyclopentyl, Cylohexyl, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlor methoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchloromethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen, unsubstituiertes oder ein- bis dreifach durch Fluor oder Chlor substituiertes Phenyl oder Phenoxy; und wobei als Naphthylsubstituenten jeweils in Frage kommen: Fluor, Chlor, Brom, Cyano, Methyl, Ethyl, n- oder i-Propyl,
$R^2$, $R^3$, $R^4$, $R^5$ und $R^6$ unabhängig voneinander für Wasserstoff oder Methyl stehen und
A für Stickstoff oder eine CH-Gruppe steht.

Insbesondere bevorzugt sind Verbindungen der Formel (I), in denen
$R^1$ für geradkettiges oder verzweigtes Alkyl mit 3 bis 7 Kohlenstoffatomen, geradkettiges oder verzweigtes Alkenyl mit 3 bis 7 Kohlenstoffatomen oder geradkettiges oder verzweigtes Alkinyl mit 3 bis 7 Kohlenstoffatomen steht;
oder für jeweils ein- bis dreifach durch Fluor, Chlor, Methoxy, Ethoxy, Methylthio, Ethylthio, Trifluormethyl, Trifluormethoxy oder Trifluormethylthio substituiertes i-Propyl oder t-Butyl steht; oder für jeweils einfach durch Cyano, Hydroximino, Methoximino, Ethoximino, Cyclopentyl, Cyclohexyl, Dioxolanyl, Dithiolanyl, Dioxanyl oder Dithianyl substituiertes Methyl, Ethyl, i-Propyl oder t-Butyl steht;
oder für jeweils geradkettiges oder verzweigtes, einfach oder zweifach durch Fluor, Chlor oder Brom substituiertes Alkenyl oder Alkinyl mit jeweils 3 bis 7 Kohlenstoffatomen steht;
oder für jeweils unsubstituiertes oder ein- bis fünffach gleich oder verschieden durch Fluor, Chlor, Brom oder Methyl substituiertes Cyclopropyl, Cyclobutyl, Cyclopentyl oder Cyclohexyl steht; oder für α-Naphthyl oder ß-Naphthyl steht; oder für unsubstituiertes oder ein- bis dreifach, gleich oder verschieden durch Fluor, Chlor, Brom, Methyl, Methoxy, Methylthio, Trifluormethyl, Trifluormethoxy, Trifluormethylthio, Difluormethyl, Difluormethoxy, Difluormethylthio, Phenyl oder Phenoxy substituiertes Phenyl steht;
oder für einen Rest der Formel

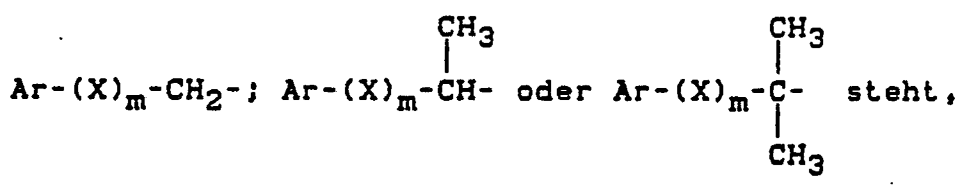
oder
steht,

wobei
Ar jeweils für unsubstituiertes oder ein- bis dreifach, gleich oder verschieden durch Fluor, Chlor, Brom, Methyl, Methoxy, Methylthio, Trifluormethyl, Trifluormethoxy, Trifluormethylthio, Difluormethyl, Difluormethoxy, Difluormethylthio, Phenyl oder Phenoxy substituiertes Phenyl steht oder für jeweils unsubstituiertes oder durch Fluor, Chlor oder Methyl substituiertes α-Naphthyl oder ß-Naphthyl steht,

X für Sauerstoff, Schwefel oder für eine der Gruppen $-CH_2-$, $-O-CH_2-$, $-S-CH_2-$, $-O-CH_2-CH_2-$ oder $-S-CH_2-CH_2-$ steht und
m für eine Zahl 0 oder 1 steht,
$R^2$, $R^3$, $R^4$, $R^5$ und $R^6$ für Wasserstoff stehen und
A für Stickstoff oder eine CH-Gruppe steht.

Bevorzugte erfindungsgemäße Verbindungen sind auch Additionsprodukte aus Säuren und denjenigen substituierten Dioxolanen der Formel (I), in denen die Sub stituenten $R^1$, $R^2$, $R^3$, $R^4$, $R^5$, $R^6$ und A die Bedeutungen haben, die bereits vorzugsweise für diese Substituenten genannt wurden, insbesondere die pflanzenverträglichen Säureadditionsprodukte.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen substituierten Dioxolanen der Formel (I), in denen die Substituenten $R^1$, $R^2$, $R^3$, $R^4$, $R^5$, $R^6$ und A die Bedeutungen haben, die bereits vorzugsweise für diese Substituenten genannt wurden, insbesondere die pflanzenverträglichen Metallkomplexe.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzenverträglichen Additionsprodukten führen. Besonders bevor zugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Im einzelnen seien außer den bei den Herstellungsbeispielen genannten Verbindungen die folgenden substituierte Dioxolane der allgemeinen Formel (I) genannt:

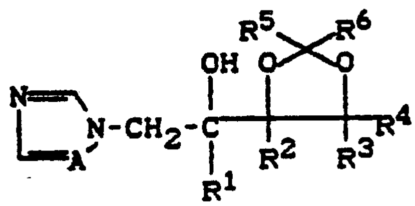

(I)

| $R^1$ | 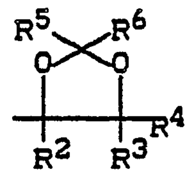 | A |
|---|---|---|
| 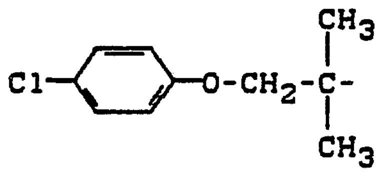 | 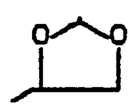 | N |
| 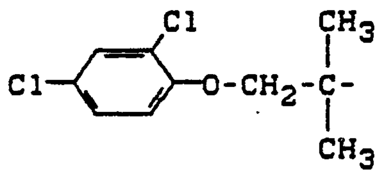 | 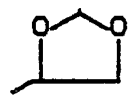 | N |
| 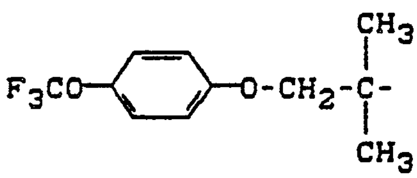 | 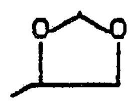 | N |
| 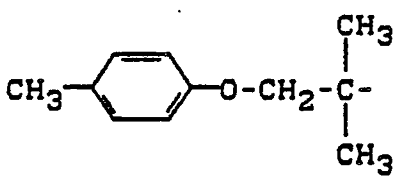 | 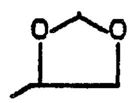 | N |
| 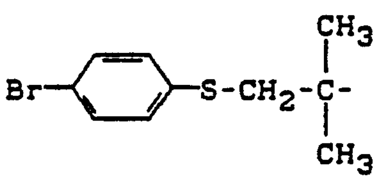 | 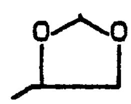 | N |
| 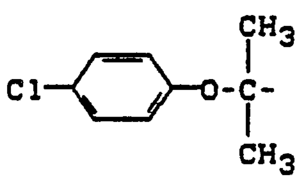 | 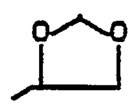 | N |
| 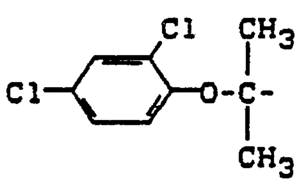 | 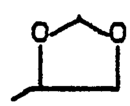 | N |

| $R^1$ | 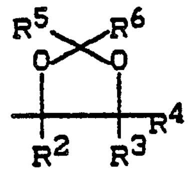 | A |
|---|---|---|
| 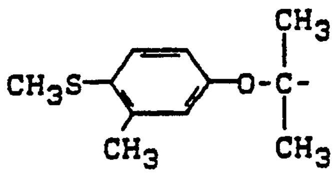 | 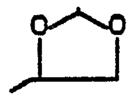 | N |
| 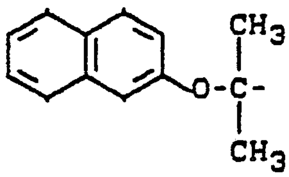 | 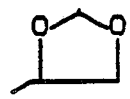 | N |
| 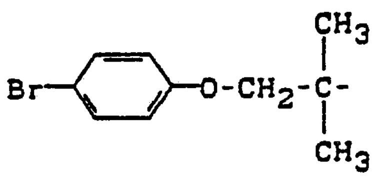 | 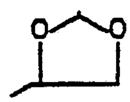 | N |
| 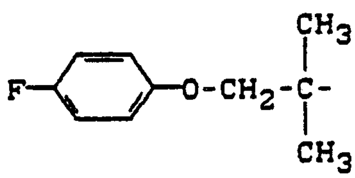 | 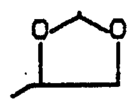 | N |
| 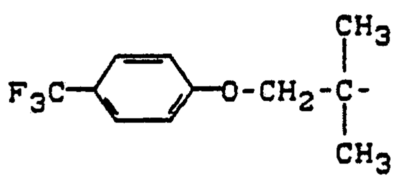 | 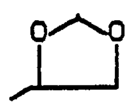 | N |
| 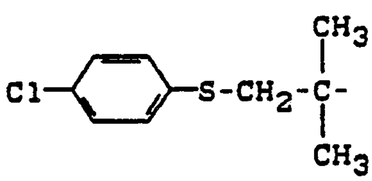 | 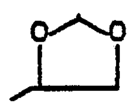 | N |

| $R^1$ | 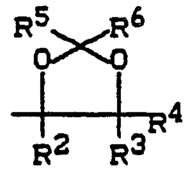 | A |
|---|---|---|
| 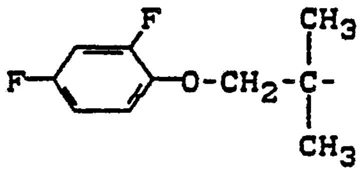 | 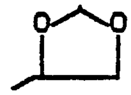 | N |
| 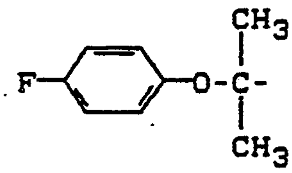 | 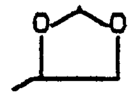 | N |
| 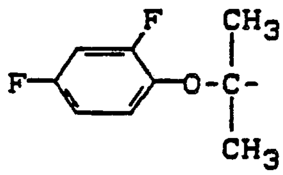 | 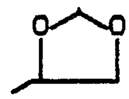 | N |
| 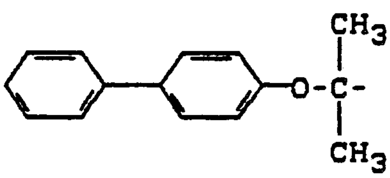 | 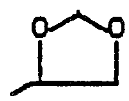 | N |
| 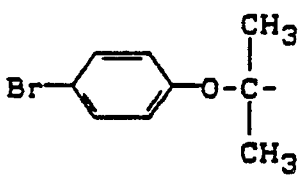 | 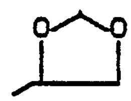 | N |
| 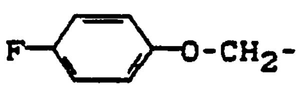 | 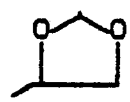 | N |
| 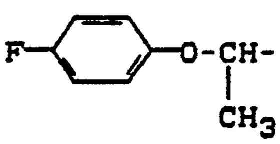 | 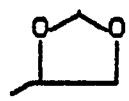 | N |

| $R^1$ | 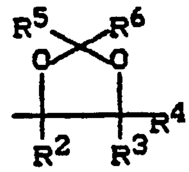 | A |
|---|---|---|
| 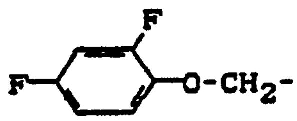 | 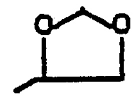 | N |
| 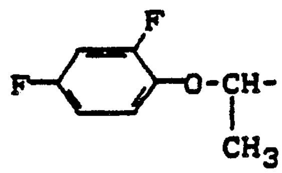 | 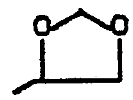 | N |
| 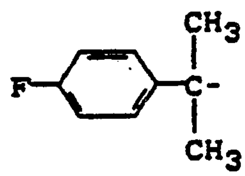 | 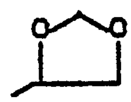 | N |
| 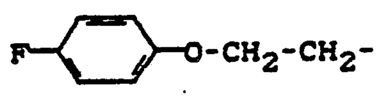 | 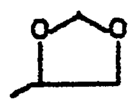 | N |
| 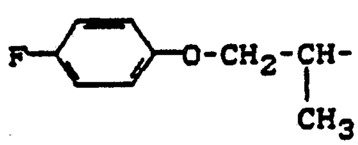 | 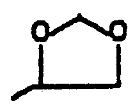 | N |
| 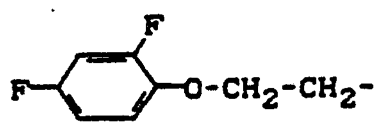 | 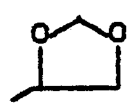 | N |
| 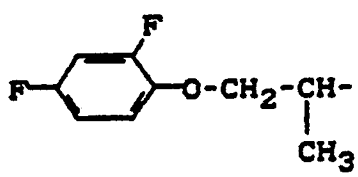 | 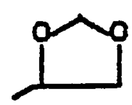 | N |
| 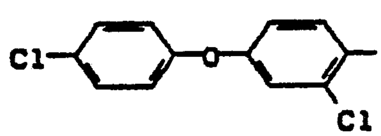 | 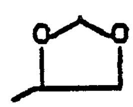 | N |

| $R^1$ | 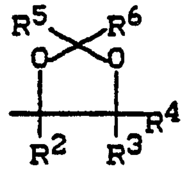 | A |
| --- | --- | --- |
| 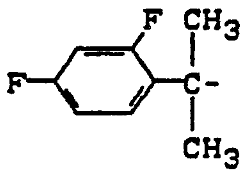 | 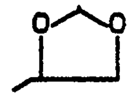 | N |
| 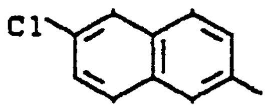 | 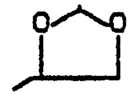 | N |
| 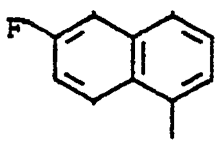 | 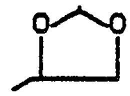 | N |
| 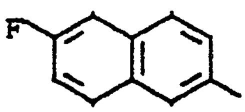 | 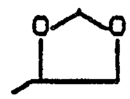 | N |
| 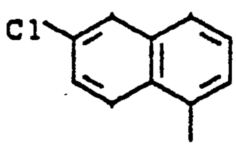 | 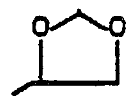 | N |
| 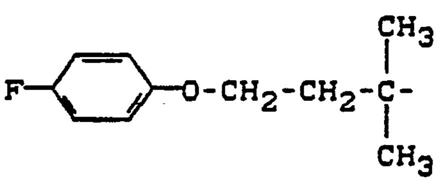 | 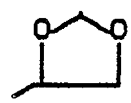 | N |
| 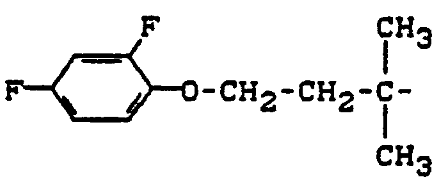 | 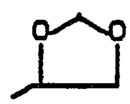 | N |
| 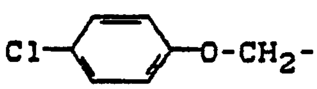 | 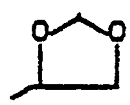 | N |

| $R^1$ | 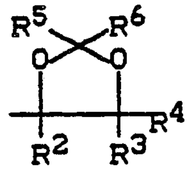 | A |
|---|---|---|
| 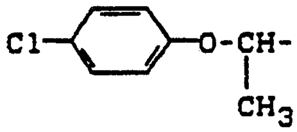 | 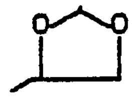 | N |
| 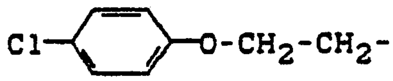 | 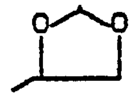 | N |
| 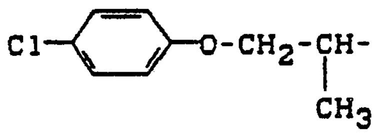 | 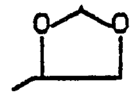 | N |
| 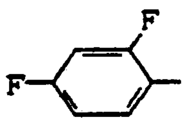 | 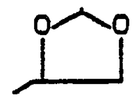 | N |
| 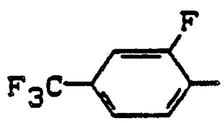 | 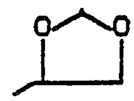 | N |
| 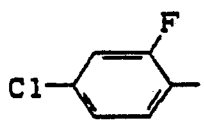 | 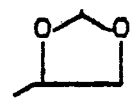 | N |
| 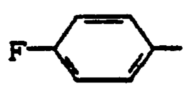 | 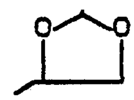 | N |
| 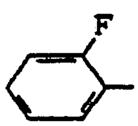 | 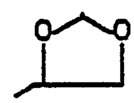 | N |

| $R^1$ | 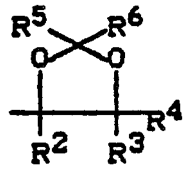 | A |
|---|---|---|
| 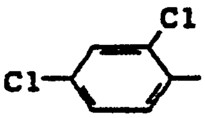 | 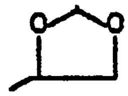 | N |
| 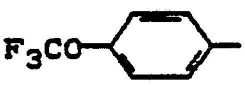 | 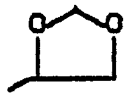 | N |
| 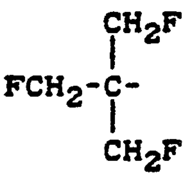 | 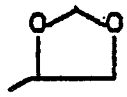 | N |
| 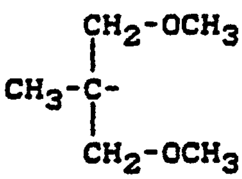 | 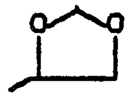 | N |
| 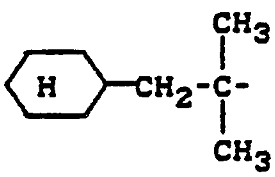 | 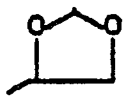 | N |
| 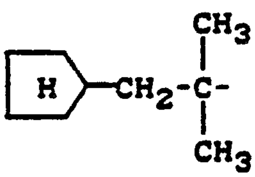 | 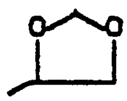 | N |
| 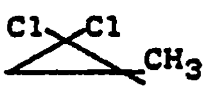 | 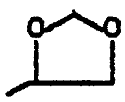 | N |
| 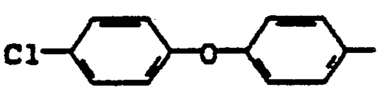 | 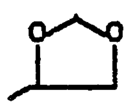 | N |

| $R^1$ | 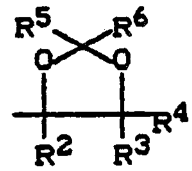 | A |
| --- | --- | --- |
| 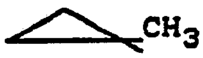 | 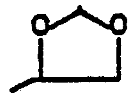 | N |
| 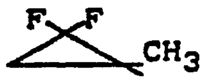 | 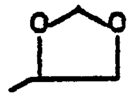 | N |
| 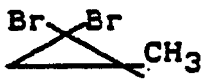 | 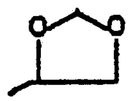 | N |
| 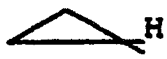 | 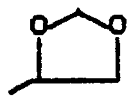 | N |
| 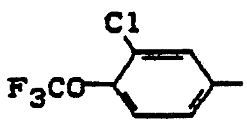 | 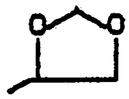 | N |
| 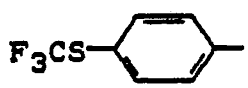 | 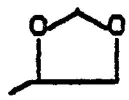 | N |
| 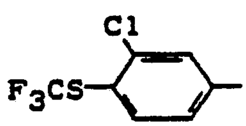 | 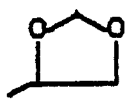 | N |
| 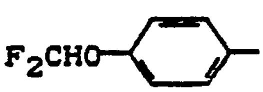 | 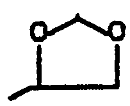 | N |
| 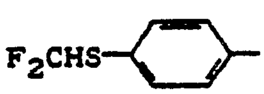 | 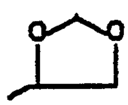 | N |
| 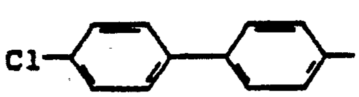 | 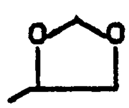 | N |

| $R^1$ | 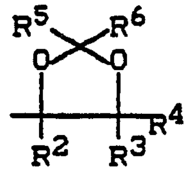 | A |
|---|---|---|
| 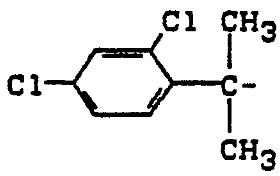 | 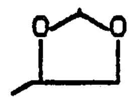 | N |
| 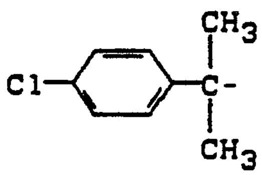 | 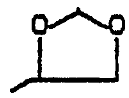 | N |
| 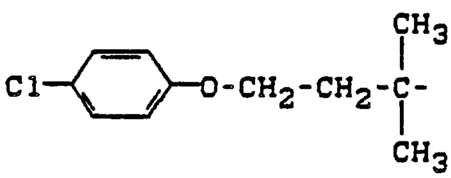 | 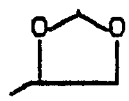 | N |
| 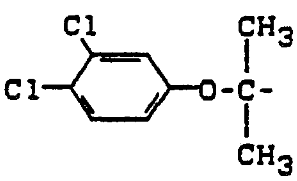 | 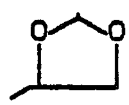 | N |
| 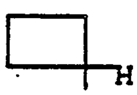 | 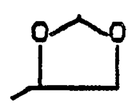 | N |
| 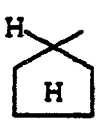 | 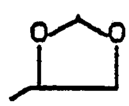 | N |
| 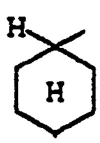 | 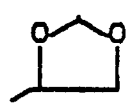 | N |

| $R^1$ | 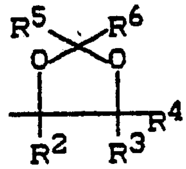 | A |
|---|---|---|
| 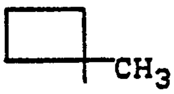 | 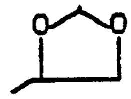 | N |
| 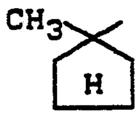 | 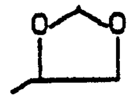 | N |
| 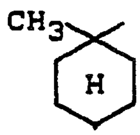 | 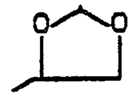 | N |
| 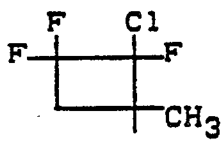 | 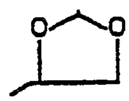 | N |
| $Cl-CH=CH-CH_2-$ | 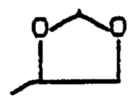 | N |
| $(CH_3)_3C-$ | 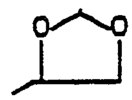 | N |
| $(CH_3)_2CH-$ | 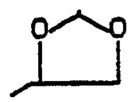 | N |
| 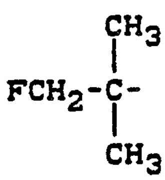 | 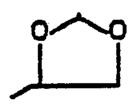 | N |

| $R^1$ | 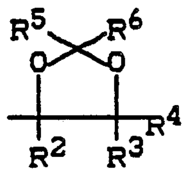 | A |
|---|---|---|
| 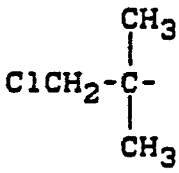 | 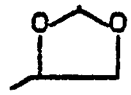 | N |
| 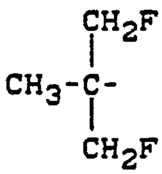 | 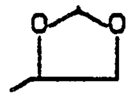 | N |
| 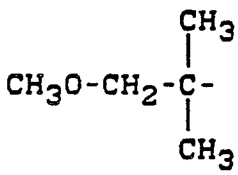 | 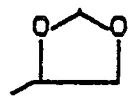 | N |
| 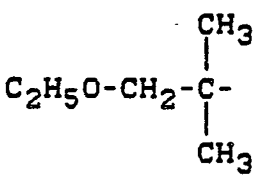 | 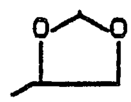 | N |
| 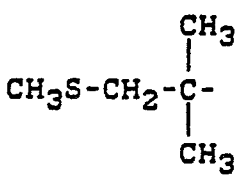 | 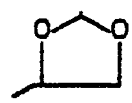 | N |
| 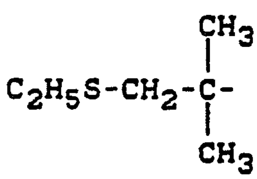 | 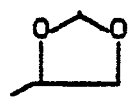 | N |

| $R^1$ | 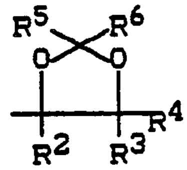 | A |
|---|---|---|
| 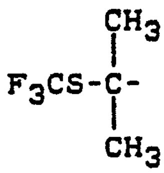 | 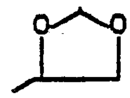 | N |
| 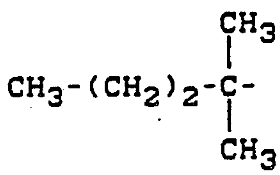 | 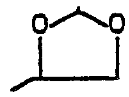 | N |
| 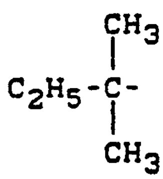 | 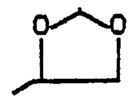 | N |
| 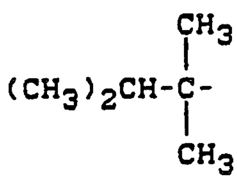 | 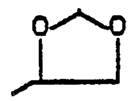 | N |
| 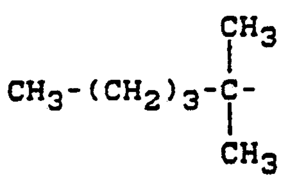 | 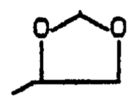 | N |
| 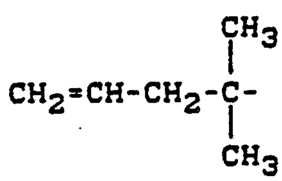 | 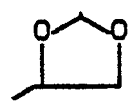 | N |
| 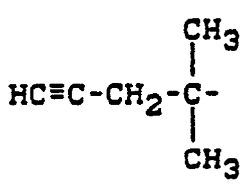 | 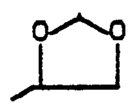 | N |

| $R^1$ | 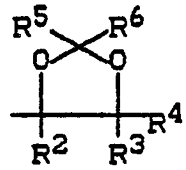 | A |
|---|---|---|
| 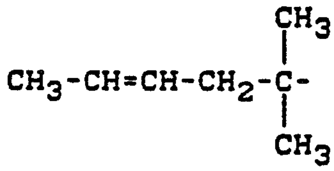 | 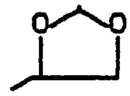 | N |
| 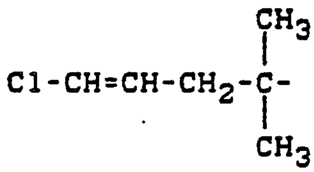 | 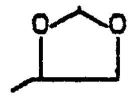 | N |
| 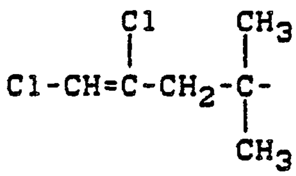 | 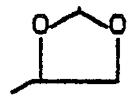 | N |
| 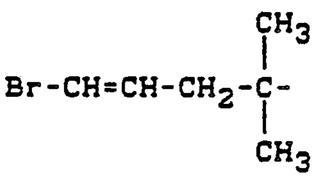 | 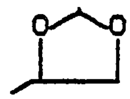 | N |
| 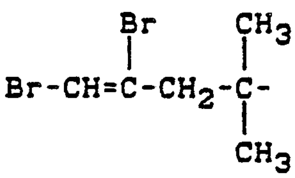 | 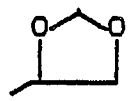 | N |
| $H_2C=CH-CH_2-$ | 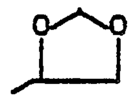 | N |
| $HC\equiv C-CH_2-$ | 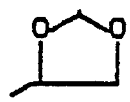 | N |

| $R^1$ | 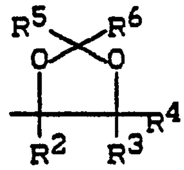 | A |
|---|---|---|
| 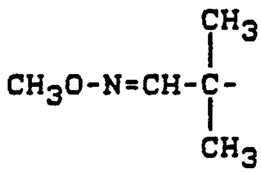 | 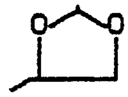 | N |
| 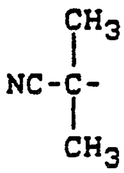 | 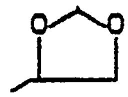 | N |
| 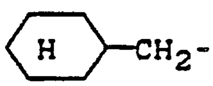 | 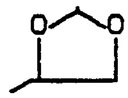 | N |
| 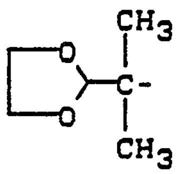 | 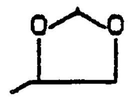 | N |
| 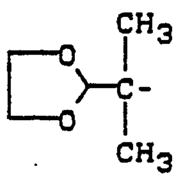 | 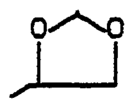 | N |
| 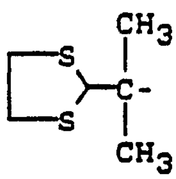 | 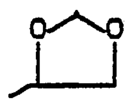 | N |
| 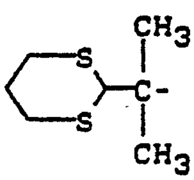 | 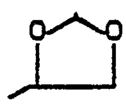 | N |

| $R^1$ | 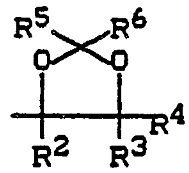 | A |
|---|---|---|
| 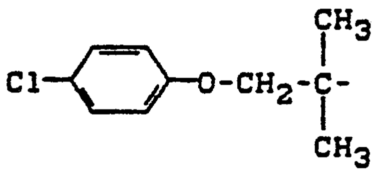 | 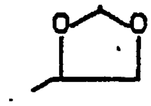 | CH |
| 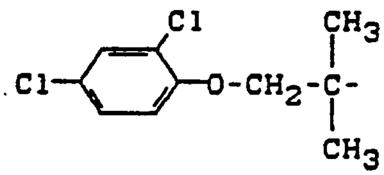 | 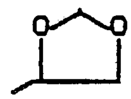 | CH |
| 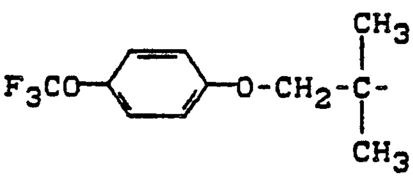 | 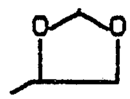 | CH |
| 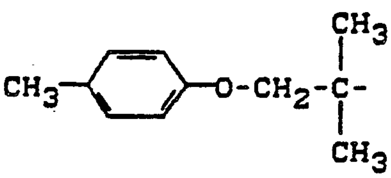 | 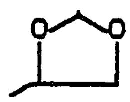 | CH |
| 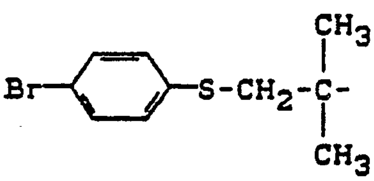 | 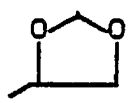 | CH |
| 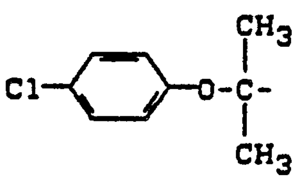 | 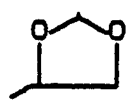 | CH |
| 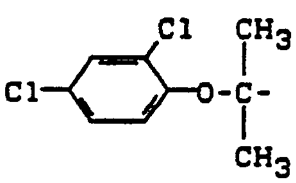 | 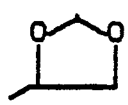 | CH |

| $R^1$ | 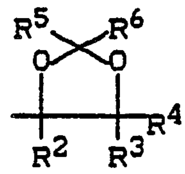 | A |
|---|---|---|
| 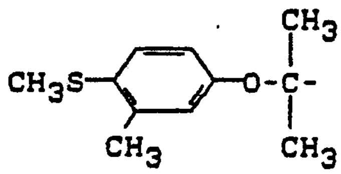 | 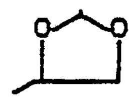 | CH |
| 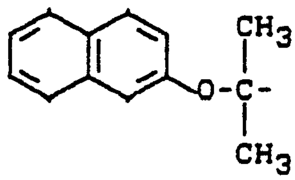 | 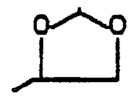 | CH |
| 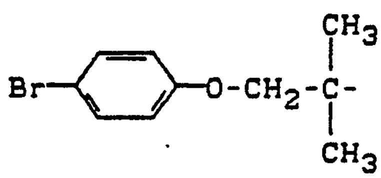 | 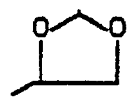 | CH |
| 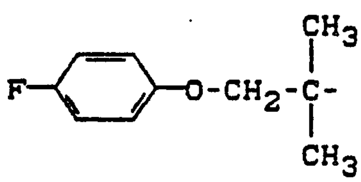 | 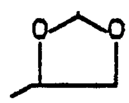 | CH |
| 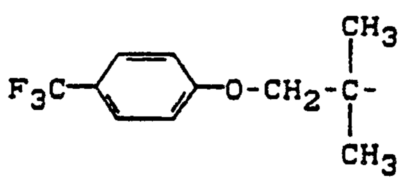 | 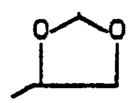 | CH |
| 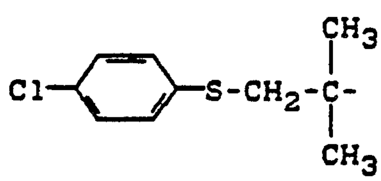 | 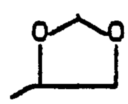 | CH |

| $R^1$ | 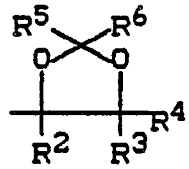 | A |
|---|---|---|
| 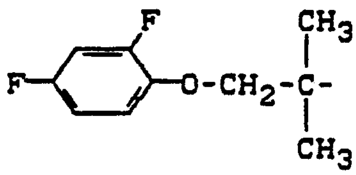 | 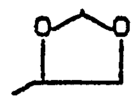 | CH |
| 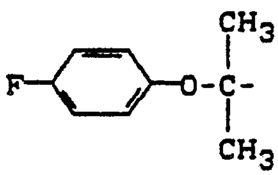 | 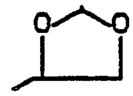 | CH |
| 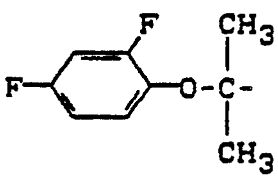 | 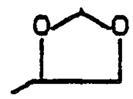 | CH |
| 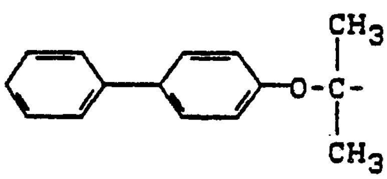 | 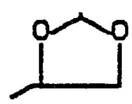 | CH |
| 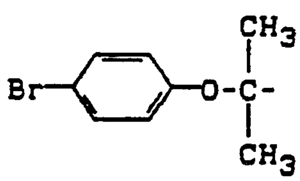 | 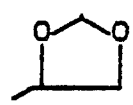 | CH |
| 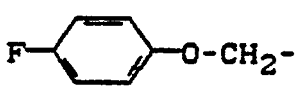 | 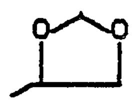 | CH |
| 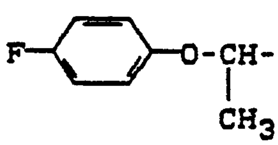 | 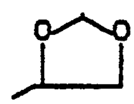 | CH |

| $R^1$ | 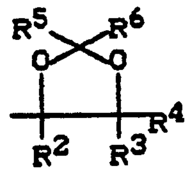 | A |
|---|---|---|
| 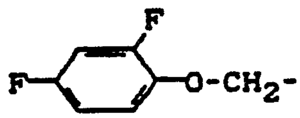 | 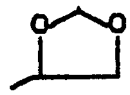 | CH |
| 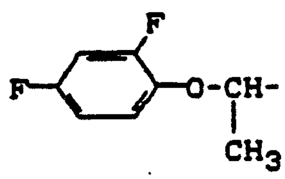 | 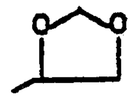 | CH |
| 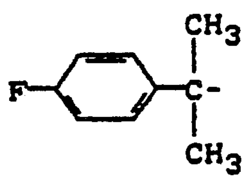 | 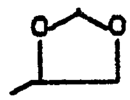 | CH |
| 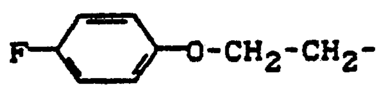 | 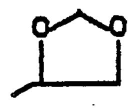 | CH |
| 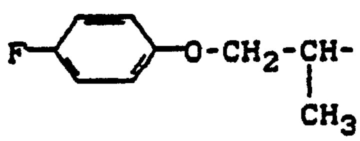 | 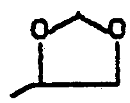 | CH |
| 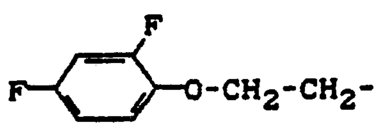 | 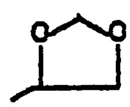 | CH |
| 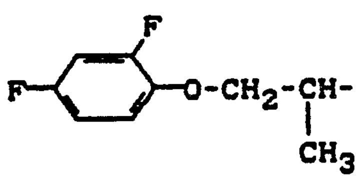 | 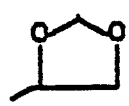 | CH |
| 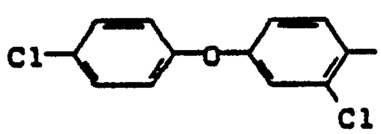 | 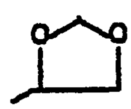 | CH |

| $R^1$ | 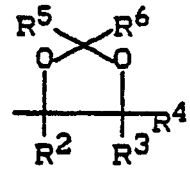 | A |
|---|---|---|
| 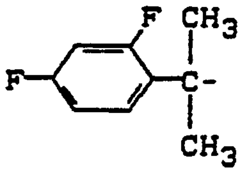 | 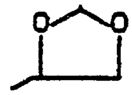 | CH |
| 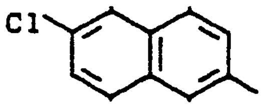 | 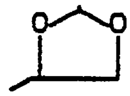 | CH |
| 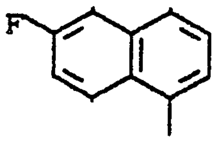 | 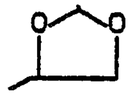 | CH |
| 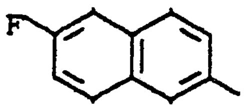 | 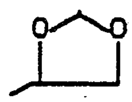 | CH |
| 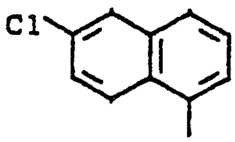 | 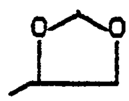 | CH |
| 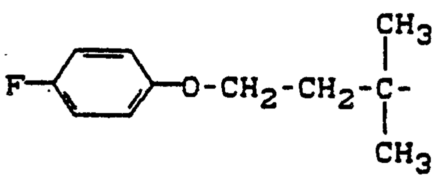 | 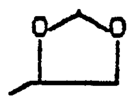 | CH |
| 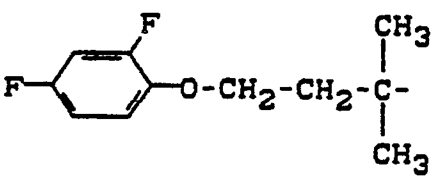 | 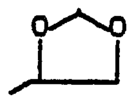 | CH |
| 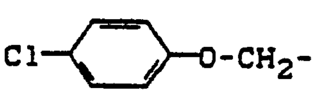 | 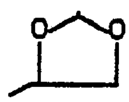 | CH |

| $R^1$ | 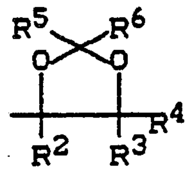 | A |
|---|---|---|
| 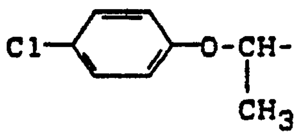 | 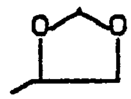 | CH |
| 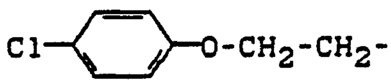 | 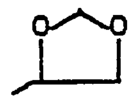 | CH |
| 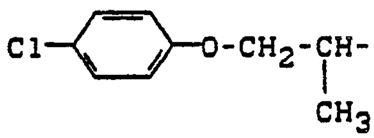 | 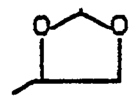 | CH |
| 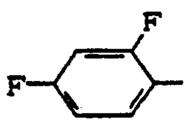 | 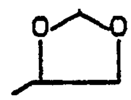 | CH |
| 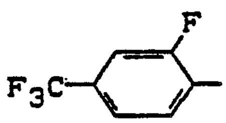 | 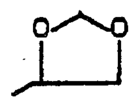 | CH |
| 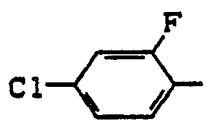 | 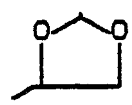 | CH |
| 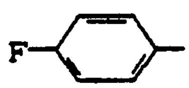 | 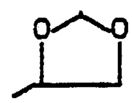 | CH |
| 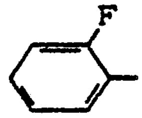 | 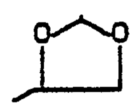 | CH |

| $R^1$ | 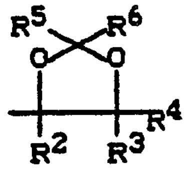 | A |
|---|---|---|
| 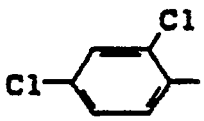 | 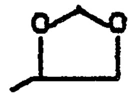 | CH |
| 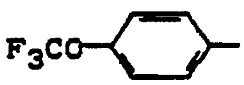 | 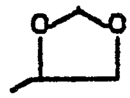 | CH |
| 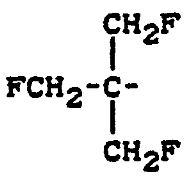 | 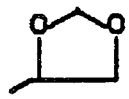 | CH |
| 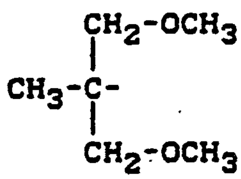 | 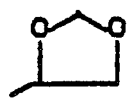 | CH |
| 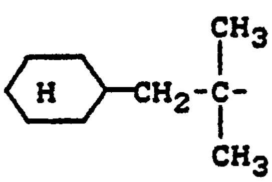 | 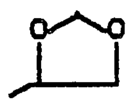 | CH |
| 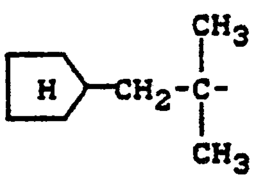 | 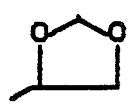 | CH |
| 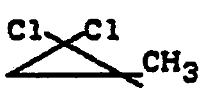 | 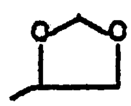 | CH |
| 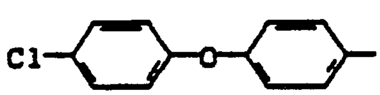 | 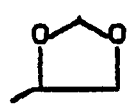 | CH |

| $R^1$ | 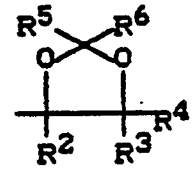 | A |
|---|---|---|
| 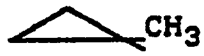 | 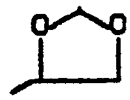 | CH |
| 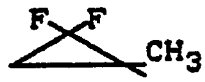 | 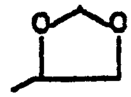 | CH |
| 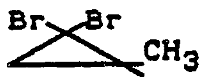 | 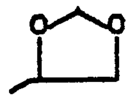 | CH |
| 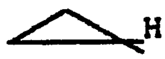 | 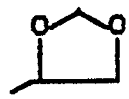 | CH |
| 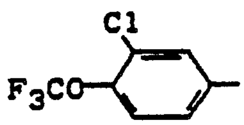 | 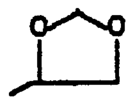 | CH |
| 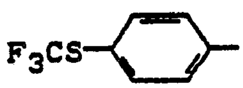 | 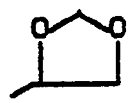 | CH |
| 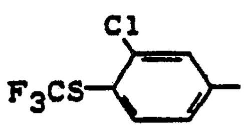 | 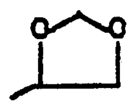 | CH |
| 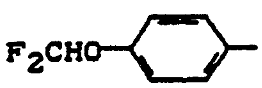 | 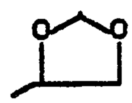 | CH |
| 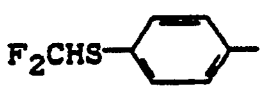 | 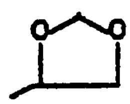 | CH |
| 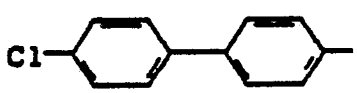 | 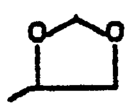 | CH |

| $R^1$ | 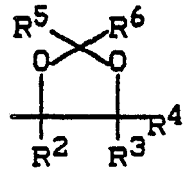 | A |
|---|---|---|
| 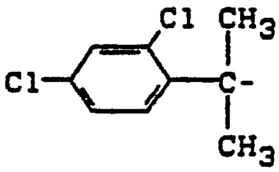 | 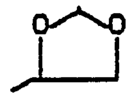 | CH |
| 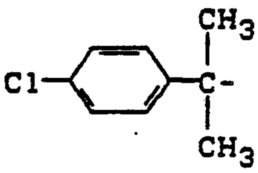 | 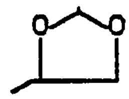 | CH |
| 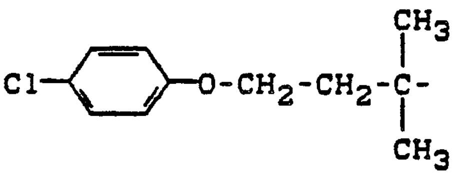 | 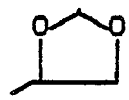 | CH |
| 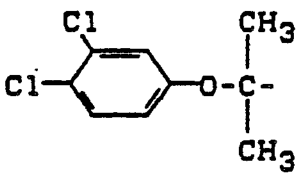 | 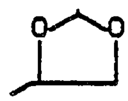 | CH |
| 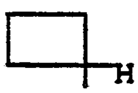 | 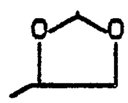 | CH |
| 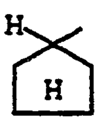 | 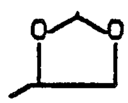 | CH |
| 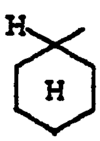 | 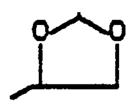 | CH |

| $R^1$ | 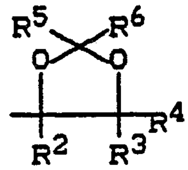 | A |
|---|---|---|
| 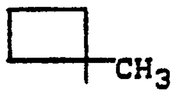 | 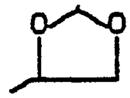 | CH |
| 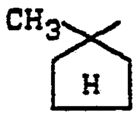 | 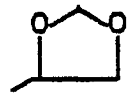 | CH |
| 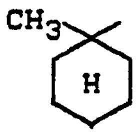 | 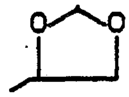 | CH |
| 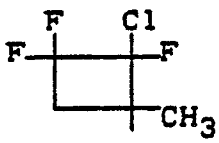 | 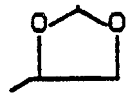 | CH |
| $Cl-CH=CH-CH_2-$ | 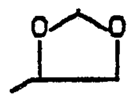 | CH |
| $(CH_3)_3C-$ | 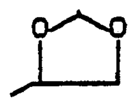 | CH |
| $(CH_3)_2CH-$ | 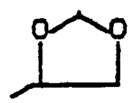 | CH |
| 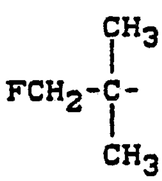 | 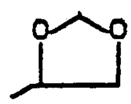 | CH |

| $R^1$ | 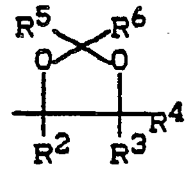 | A |
|---|---|---|
| 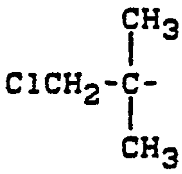 | 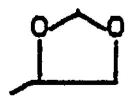 | CH |
| 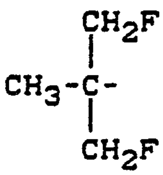 | 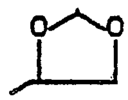 | CH |
| 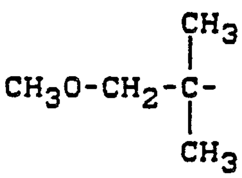 | 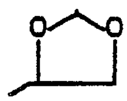 | CH |
| 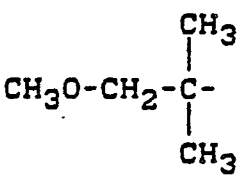 | 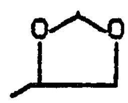 | CH |
| 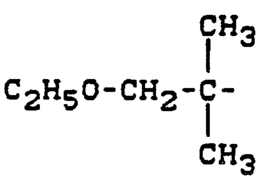 | 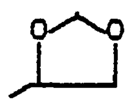 | CH |
| 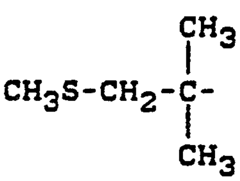 | 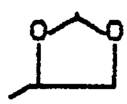 | CH |
| 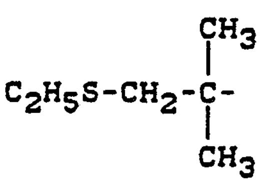 | 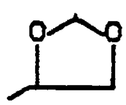 | CH |

| $R^1$ | 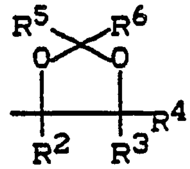 | A |
|---|---|---|
| 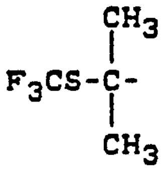 | 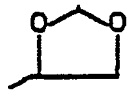 | CH |
| 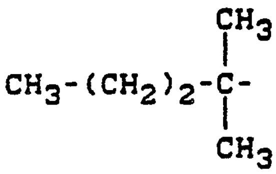 | 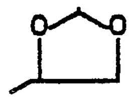 | CH |
| 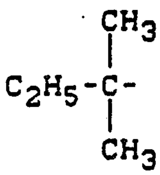 | 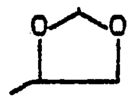 | CH |
| 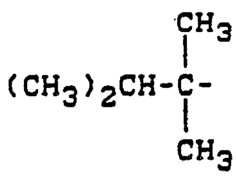 | 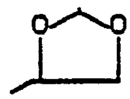 | CH |
| 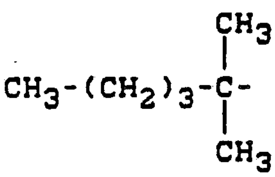 | 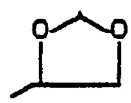 | CH |
| 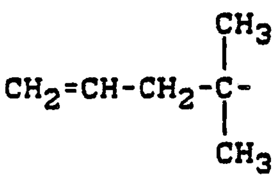 | 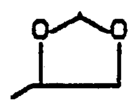 | CH |
| 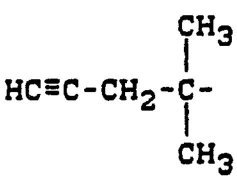 | 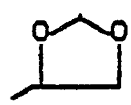 | CH |

| $R^1$ | 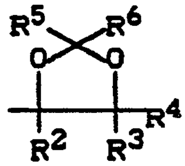 | A |
|---|---|---|
| 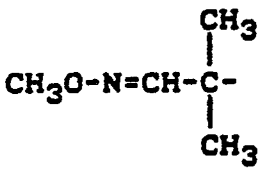 | 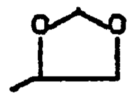 | CH |
| 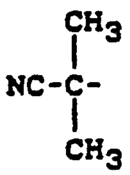 | 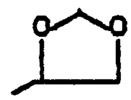 | CH |
| 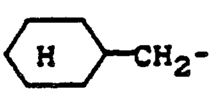 | 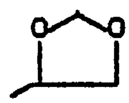 | CH |
| 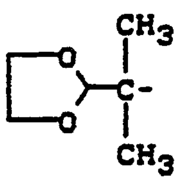 | 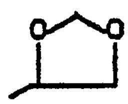 | CH |
| 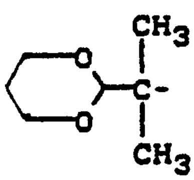 | 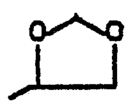 | CH |
| 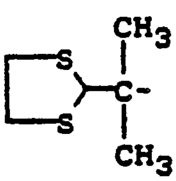 | 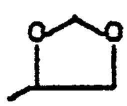 | CH |
| 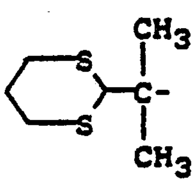 | 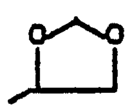 | CH |

Verwendet man beispielsweise 2-t-Butyl-2-(2,2-dimethyl-1,3-dioxolan-4-yl)-oxiran und 1,2,4-Triazol als Ausgangsstoffe, so läßt sich der Reaktionsablauf des erfindungsgemäßen Verfahrens durch das folgende Formelschema darstellen:

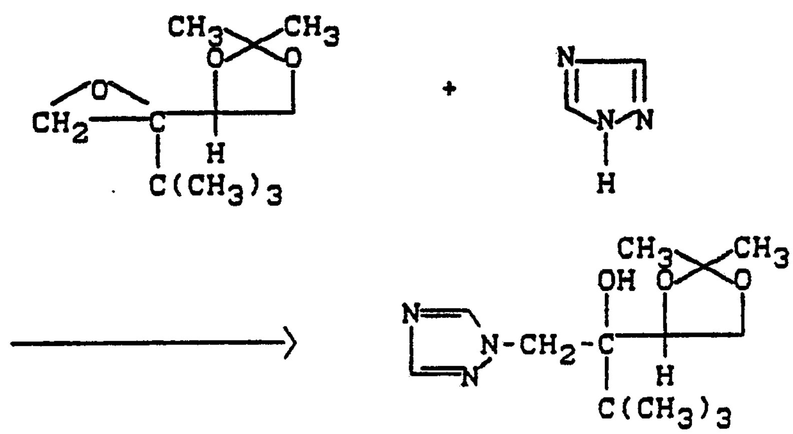

Die zur Durchführung des erfindungsgemäßen Verfahrens als Ausgangsstoffe benötigten Oxiranyldioxolane sind durch die Formel (II) allgemein definiert. In dieser Formel (II) stehen $R^1$, $R^2$, $R^3$, $R^4$, $R^5$ und $R^6$ vorzugsweise für diejenigen Reste, die bereits im Zusammenhang mit der Beschreibung der erfindungsgemäßen Stoffe der Formel (I) als bevorzugt für diese Substituenten genannt wurden.

Die Oxiranyldioxolane der Formel (II) sind teilweise bekannt (vgl. z.B. Acta chem. Scand. B 34, 41-45, [1980]; Tetrahedron 36, 3101-3105 [1980]; Tetrahdron Lett. 27, 69-70 [1986]; JP 62/26 280).

Noch nicht bekannt und ebenfalls Gegenstand der Erfindung sind Oxiranyldioxolane der Formel (IIa),

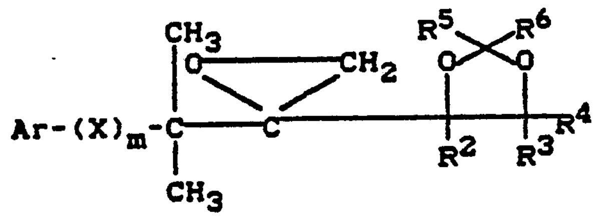

(IIa)

in welcher
Ar für gegebenenfalls substituiertes Aryl steht,
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$ oder $-S(O)_n-CH_2-CH_2-$ steht,
m für eine Zahl 0 oder 1 steht,
n jeweils für eine Zahl 0, 1 oder 2 steht und
$R^2$, $R^3$, $R^4$, $R^5$ und $R^6$ die oben angegebene Bedeutung haben.

Bevorzugt sind Oxiranyldioxolane der Formel (IIa), in denen
Ar für unsubstituiertes oder einfach oder mehrfach, gleich oder verschieden substituiertes Phenyl steht, wobei als Substituenten in Frage kommen: Halogen, Cyano, Nitro, geradkettiges oder verzweig tes Alkyl, geradkettiges oder verzweigtes Alkoxy, geradkettiges oder verzweigtes Alkylthio, geradkettiges oder verzweigtes Alkylsulfinyl oder geradkettiges oder verzweigtes Alkylsulfonyl mit jeweils 1 bis 4 Kohlenstoffatomen, geradkettiges oder verzweigtes, zweifach verknüpftes Alkandiyl mit 1 bis 10 Kohlenstoffatomen, Cycloalkyl mit 3 bis 7 Kohlenstoffatomen, geradkettiges oder verzweigtes Halogenalkyl, geradkettiges oder verzweigtes Halogenalkoxy, geradkettiges oder verzweigtes Halogenalkylthio, geradkettiges oder verzweigtes Halogenalkylsulfinyl oder geradkettiges oder verzweigtes Halogenalkylsulfonyl mit jeweils 1 bis 4 Kohlenstoffatomen und 1 bis 9 gleichen oder verschiedenen Halogenatomen, geradkettiges oder verzweigtes Alkoxycarbonyl oder geradkettiges oder verzweigtes Alkoximinoalkyl mit jeweils 1 bis 4 Kohlenstoffatomen in den einzelnen Alkylteilen, unsubstituiertes oder einfach oder mehrfach, gleich oder verschieden durch Halogen substituiertes, zweifach verknüpftes Dioxyalkylen mit 1 bis 6 Kohlenstoffatomen oder jeweils unsubstituiertes oder einfach oder mehrfach, gleich oder verschieden durch Halogen substituiertes Phenyl oder Phenoxy; oder für unsubstituiertes oder einfach oder mehrfach, gleich oder verschieden substituiertes Naphthyl steht, wobei als Substituenten in Frage kommen: Halogen, Cyano oder geradkettiges oder verzweigtes Alkyl mit 1 bis 4 Kohlenstoffatomen;
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$ oder $-S(O)_n-CH_2-CH_2-$ steht,
m für eine Zahl 0 oder 1 steht,

n jeweils für eine Zahl 0, 1 oder 2 steht und
$R^2$, $R^3$, $R^4$, $R^5$ und $R^6$ die oben angegebene Bedeutung haben.

Besonders bevorzugt sind Oxiranyldioxolane der Formel (IIa), in denen
Ar für unsubstituiertes oder ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Propan-1,3-diyl, Butan-1,4-diyl, Pentan-1,5-diyl, Dimethylpropan-1,3-diyl, Tetramethylpropan-1,3-diyl, Dimethylbutan-1,4-diyl, Tetramethylbutan-1,4-diyl, Cyclopropyl, Cyclobutyl, Cyclopentyl, Cylohexyl, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen, jeweils unsubstituiertes oder ein- bis dreifach durch Fluor oder Chlor substituiertes Phenyl oder Phenoxy;
oder für unsubstituiertes oder ein- bis dreifach, gleich oder verschieden substituiertes α-Naphthyl oder ß-Naphthyl steht, wobei als Substituenten jeweils infrage kommen: Fluor, Chlor, Brom, Cyano, Methyl, Ethyl, n- oder i-Propyl,
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$ oder $-S(O)_n-CH_2-CH_2-$ steht,
m für eine Zahl 0 oder 1 steht,
n jeweils für eine Zahl 0, 1 oder 2 steht und
$R^2$, $R^3$, $R^4$, $R^5$ und $R^6$ die oben angegebene Bedeutung haben.

Ganz besonders bevorzugt sind Oxiranyldioxolane der Formel (IIa), in denen
Ar für unsubstituiertes oder ein- bis dreifach, gleich oder verschieden durch Fluor, Chlor, Brom, Methyl, Methoxy, Methylthio, Trifluormethyl, Trifluormethoxy, Trifluormethylthio, Difluormethyl, Difluormethoxy, Difluormethylthio, Phenyl oder Phenoxy substituiertes Phenyl steht oder für jeweils unsubstituiertes oder einfach durch Fluor, Chlor oder Methyl substituiertes α-Naphthyl oder ß-Naphthyl steht,
X für Sauerstoff, Schwefel oder für eine der Gruppen $-CH_2-$; $-O-CH_2-$; $-S-CH_2-$; $-O-CH_2-CH_2-$ oder $-S-CH_2-CH_2-$ steht,
m für eine Zahl 0 oder 1 steht und
$R^2$, $R^3$, $R^4$, $R^5$ und $R^6$ die oben angegebene Bedeutung haben.

Man erhält diese neuen Oxiranyldioxolane der Formel (IIa) in Analogie zur Herstellung der bekannten Oxi ranyldioxolane der Formel (II), wenn man Vinylketone der Formel (IVa),

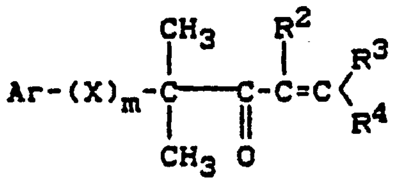

(IVa)

in welcher
Ar, X, $R^2$, $R^3$, $R^4$ und m die bei der Formel (IIa) angegebene Bedeutung haben,
mit einem Oxidationsmittel wie beispielsweise Peressigsäure oder m-Chlorperbenzoesäure gegebenenfalls in Gegenwart eines Verdünnungsmittels wie beispielsweise Dichlormethan, Dichlorbenzol, Toluol oder Essigsäure bei Temperaturen zwischen 10 °C und 60 °C epoxidiert und dann die so erhältlichen Oxiranylketone der Formel (Va),

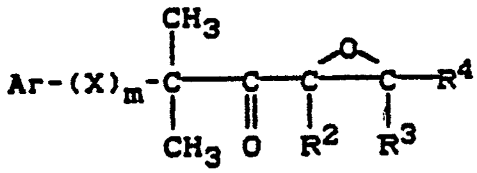

(Va)

in welcher
Ar, X, $R^2$, $R^3$, $R^4$ und m die bei der Formel (IIa) angegebene Bedeutung haben,
in einer 2. Stufe mit Aldehyden oder Ketonen der Formel (VI),

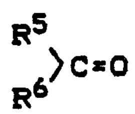

(VI)

in welcher
$R^5$ und $R^6$ die oben angegebene Bedeutung haben,
gegebenenfalls in Gegenwart eines Katalysators wie beispielsweise Zinntetrachlorid und gegebenenfalls in Gegenwart eines Verdünnungsmittels wie beispielsweise Tetrachlorkohlenstoff bei Temperaturen zwischen - 80 °C und + 50 °C umsetzt (vgl. z.B. Angew. Chem. Int. Ed. Engl. 21, 449 [1982]) und dann die so erhältlichen Dioxolanylketone der Formel (VIIa),

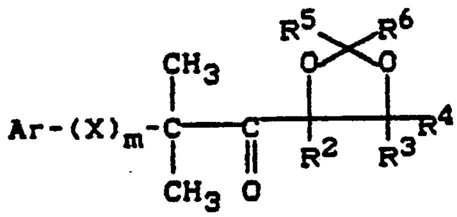

(VIIa)

in welcher
Ar, X, $R^2$, $R^3$, $R^4$, $R^5$, $R^6$ und m die bei der Formel (IIa) angegebene Bedeutung haben,
entweder mit Dimethyloxosulfoniummethylid der Formel (VIII)

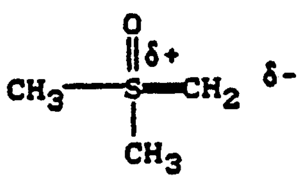

(VIII)

in bekannter Weise in Gegenwart eines Verdünnungsmittels wie beispielsweise Dimethylsulfoxid, bei Temperaturen zwischen 20 °C und 80 °C umsetzt (vgl. z. B. J. Amer. Chem. Soc. 87, 1363 - 1364 [1965]), oder mit Trimethylsulfoniummethylsulfat der Formel (IX)

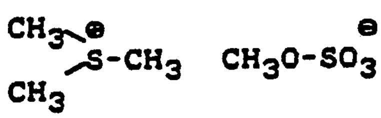

(IX)

ebenfalls in bekannter Weise in Gegenwart eines Verdünnungsmittels, wie beispielsweise Acetonitril und in Gegenwart einer Base wie beispielsweise Natriummethylat, bei Temperaturen zwischen 0 °C und 60 °C umsetzt (vgl. z.B. Heterocycles 8, 397 [1977]).

Die so erhältlichen Oxiranyldioxolane der Formel (II) können gegebenenfalls direkt aus dem Reaktionsgemisch heraus ohne Isolierung nach dem erfindungsgemäßen Verfahren weiter umgesetzt werden.

Vinylketone der Formel (IVa) sind bekannt oder erhältlich in Analogie zu bekannten Verfahren (vgl. z.B. EP 6718; DE-OS 29 22 070; Chem. Lett. 1987, 1283-1286; J. Amer. chem. Soc. 108, 4568-4580 [1986]; J.

org. Chem. 51, 2389-2391 [1986] oder An. Quim. 75, 707-711 [1979] bzw. CA 92: 75 727a).

Oxiranylketone der Formel (Va) und Dioxolanylketone der Formel (VIIa) sind noch nicht bekannt und sind ebenfalls Gegenstand der Erfindung.

Dioxolanylketone der Formel (VII),

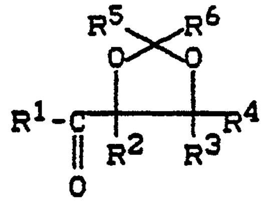

(VII)

in welcher
$R^1$, $R^2$, $R^3$, $R^4$, $R^5$ und $R^6$ die oben angegebenen Bedeutung haben,
sind teilweise bekannt (vgl. z.B. J. org. Chem. 33, 2473-2477 [1968];Acta chem. Scand. B 34, 41-45 [1980]; Tetrahedron 36, 3101-3105 [1980]; J. org. Chem. 47, 3289-3296 [1982]; Tetrahedron Lett. 23, 4369-4370 [1982]; Synthesis 1986 60-61; Synth. Commun. 16, 1517-1522 [1986]; Tetrahedron Lett. 28, 383-386 [1987]-), oder erhältlich in Analogie zu bekannten Verfahren, beispielsweise wenn man Dioxolanylcarbinole der Formel (X),

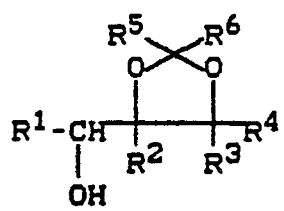

(X)

in welcher
$R^1$, $R^2$, $R^3$, $R^4$, $R^5$ und $R^6$ die oben angegebene Bedeutung haben,
mit üblichen Oxidationsmitteln wie beispielsweise Chromtrioxid in Gegenwart von Pyridin und Chlorwasserstoff sowie gegebenenfalls in Gegenwart eines Verdünnungsmittels wie beispielsweise Dichlormethan bei Temperaturen zwischen - 20 °C und + 80 °C oxidiert (vgl. z.B. Tetrahedron Lett. 28, 383-386 [1987]).

Dioxolanylcarbinole der Formel (X) sind bekannt oder erhältlich in Analogie zu bekannten Verfahren (vgl. z.B. Tetrahedron Lett. 26, 5759-5762 [1985]).

Dioxolanylketone der Formel (VIIb),

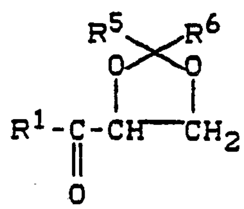

(VIIb)

in welcher
$R^1$, $R^5$ und $R^6$ die oben angegebene Bedeutung haben,
erhält man alternativ auch, wenn man Halogenmethylketone der Formel (XI),

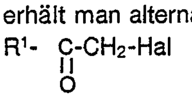

(XI)

in welcher
$R^1$ die oben angegebene Bedeutung hat und
Hal für Halogen, insbesondere für Chlor oder Brom steht,
mit Formaldehyd gegebenenfalls in Gegenwart eines Verdünnungsmittels wie beispielsweise Methanol, Tetrahydrofuran oder Dioxan und gegebenenfalls in Gegenwart eines Reaktionshilfsmittels wie beispielsweise Natriummethylat oder Natronlauge bei Temperaturen zwischen 0 °C und 120 °C umsetzt, wobei in Abhängigkeit von Reaktionsdauer, Reaktionstemperatur und Konzentration an Reaktionshilfsmittel entweder

α-Halogenketone der Formel (XII),

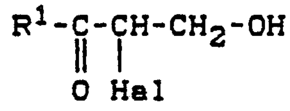 (XII)

in welcher
$R^1$ und Hal die oben angegebene Bedeutung haben,
oder Oxiranylketone der Formel (Vb),

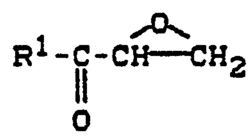 (Vb)

in welcher
$R^1$ die oben angegebene Bedeutung hat,
entstehen, und wenn man diese α-Halogenketone der Formel (XII) oder die Oxiranylketone der Formel (Vb) oder ein Gemisch aus Verbindungen dieser Art in einer 2. Stufe mit Aldehyden oder Ketonen der Formel (VI),

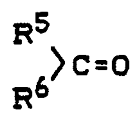 (VI)

in welcher
$R^5$ und $R^6$ die oben angegebene Bedeutung haben,
gegebenenfalls in Gegenwart eines Verdünnungsmittels wie beispielsweise Tetrachlorkohlenstoff und gegebenenfalls in Gegenwart eines Katalysators wie beispielsweise Zinntetrachlorid und gegebenenfalls in Gegenwart eines Säurebindemittels wie beispielsweise Natriummethylat oder Natriumhydroxid bei Temperaturen zwischen - 80 °C und + 80 °C umsetzt (vgl. z.B. Angew. Chem. Int. Ed. Engl. 21, 449 [1982]).

Aldehyde und Ketone der Formel (VI) sind allgemein bekannte Verbindungen der organischen Chemie.

Halogenmethylketone der Formel (XI) sind ebenfalls allgemein bekannte Verbindungen der organischen Chemie oder erhältlich in Analogie zu allgemein bekannten Verfahren.

Oxiranylketone der Formel (Vb) sind teilweise bekannt (vgl. z.B. Can. J. Chem. 62, 2429-2434 [1984]; Tetrahedron 40, 1381-1390 [1984]; Chem. Lett. 1982, 1601-1604; J. org. Chem. 45, 3407-3413 [1980]; J. org. Chem. 43, 1323-1327 [1978]; Chem. Ber. 108, 2391-2396 [1975]; J. org. Chem. 39, 388-393 [1974].

α-Halogenketone der Formel (XII) sind ebenfalls teilweise bekannt (vgl. z.B. Tetrahedron Lett. 28, 383-386 [1987]; Synth. Commun. 16, 1517-1522 [1986]; Synthesis 1986, 60-61; Tetrahedron Lett. 23, 4369-4370 [1982]; J. org Chem. 47, 3289-3296 [1982]; Tetrahedron 36, 3101-3105 [1980]; Acta chem. Scand B 34, 41-45 [1980]; J. org. Chem. 33 , 2473-2477 [1968]).

Noch nicht bekannt sind α-Halogenketone der Formel (XIIa),

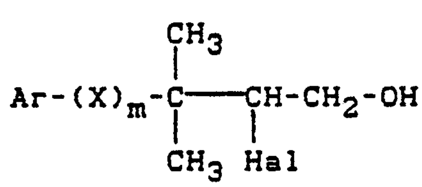 (XIIa)

in welcher
Hal für Halogen, insbesondere für Chlor oder Brom steht und
Ar, X und m die oben angegebene Bedeutung haben.

Man erhält sie in Analogie zum oben angegebenen allgemeinen Verfahren, wenn man Halogenmethylketone der Formel (XIa),

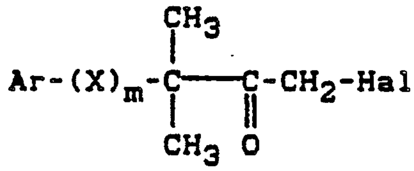

(XIa)

in welcher
Ar, X, Hal und m die oben angegebene Bedeutung haben,
mit Formaldehyd gegebenenfalls in Gegenwart eines Verdünnungsmittels wie beispielsweise Methanol, Ethanol, Tetrahydrofuran oder Dioxan und gegebenenfalls in Gegenwart eines Reaktionshilfsmittels wie beispielsweise Natriummethylat bei Temperaturen zwischen 0 °C und 60 °C umsetzt.

Halogenmethylketone der Formel (XIa) sind bekannt oder erhältlich in Analogie zu bekannten Verfahren (vgl. z.B. DE-OS 26 32 603; DE-OS 30 21 516; EP 54 865).

Dioxolanylketone der Formel (VIIc)

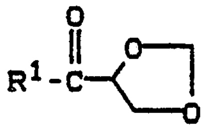

(VIIc)

in welcher
$R^1$ die oben angegebene Bedeutung hat,
erhält man alternativ auch, wenn man Halogenmethylketone der Formel (XI),

$R^1$- C-$CH_2$-Hal (XI)
||
O

in welcher
$R^1$ die oben angegebene Bedeutung hat und
Hal für Halogen, insbesondere für Chlor oder Brom steht,
mit mindestens zwei Äquivalenten Formaldehyd gegebenenfalls in Gegenwart eines Verdünnungsmittels wie beispielsweise Methanol oder Tetrahydrofuran und gegebenenfalls in Gegenwart eines Säurebindemittels wie beispielsweise Natriummethylat oder Natriumhydroxid bei Temperaturen zwischen 20 °C und 80 °C umsetzt, wobei intermediär auftretende α-Halogenketone der Formel (XII) nicht isoliert werden.

Die zur Durchführung des erfindungsgemäßen Verfahrens weiterhin als Ausgangsstoffe benötigten Azole sind durch die Formel (III) allgemein definiert. In dieser Formel (III) steht A vorzugsweise für diejenigen Reste, die bereits im Zusammenhang mit der Beschreibung der erfindungsgemäßen Stoffe der Formel (I) als bevorzugt für diesen Substituenten genannt wurden.

Azole der Formel (III) sind allgemein bekannte Verbindungen der organischen Chemie.

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Alkohole, wie Methanol, Ethanol, Propanol, Butanol, Methoxyethanol oder Ethoxyethanol.

Das erfindungsgemäße Verfahren wird vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethyl amin, N,N-Dimethylanilin, Pyridin, N,N-Dimethylaminopyridin, Diazabicyclooctan (DABCO), Diazabicyclononen (DBN) oder Diazabicycloundecen (DBU).

Darüberhinaus kann es gegebenenfalls von Vorteil sein katalytische Mengen eines üblichen Radikalbildners wie beispielsweise α,α′-Azodiisobutyronitril (AIBN) als Reaktionshilfsmittel einzusetzen.

Die Reaktionstemperaturen können bei der Durchführung des erfindungsgemäßen Verfahrens in einem

größeren Bereich variiert werden. Im allgemeinen arbeitet man bei Temperaturen zwischen 0 °C und 200 °C, vorzugsweise bei Temperaturen zwischen 60 °C und 150 °C.

Bei der Durchführung des erfindungsgemäßen Verfahrens arbeitet man im allgemeinen unter Normaldruck. Es ist jedoch auch möglich, unter erhöhtem oder vermindertem Druck zu arbeiten.

Zur Durchführung des erfindungsgemäßen Verfahrens setzt man pro Mol an Oxiranyldioxolan der Formel (II) im allgemeinen 1.0 bis 5.0 Mol, vorzugsweise 1.0 bis 1.2 Mol an Azol der Formel (III) und gegebenenfalls 1.0 bis 5.0 Mol, vorzugsweise 1.0 bis 2.0 Mol an Reaktionshilfsmittel ein.
Die Reaktionsdurchführung, Aufarbeitung und Isolierung der Reaktionsprodukte erfolgt nach allgemein üblichen Methoden (vgl. auch die Herstellungsbeispiele).

Die Säureadditionssalze der Verbindungen der Formel (I) können in einfacher Weise nach üblichen Salzbildungsmethoden, wie z.B durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die Metallsalzkomplexe von Verbindungen der Formel (I) können in einfacher Weise nach üblichen Verfahren erhalten werden, so. z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren isolieren und gegebenenfalls durch Umkristallisation reinigen.

Darüberhinaus sind die erfindungsgemäßen substituierten Dioxolane der Formel (I) als interessante Zwischenprodukte zur Herstellung von weiteren Wirkstoffen verwendbar.
Sie können beispielsweise an der Hydroxygruppe durch Alkylierung oder Acylierung mit üblichen Alkylhalogeniden oder Alkylsulfaten oder mit Acylhalogeniden oder Carbamoylhalogeniden umgesetzt werden zu entsprechenden Ethern, Estern, Carbonaten oder Carbamaten, welche ebenfalls gute Wirksamkeit als Fungizide besitzen.

Die erfindungsgemäßen Wirkstoffe weisen eine starke Wirkung gegen unerwünschte Mikroorganismen auf. Die Wirkstoffe sind vorzugsweise für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosphorium);
Cochliobolus-Arten, wie beispielsweise Cochliobolus sativus
(Konidienform: Drechslera, Syn: Helminthosphorium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Kon zentrationen erlaubt eine Behandlung zon oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen der Erreger des echten Getreidemehltaus (Erysiphe graminis) oder gegen den Erreger des Getreidebraunrostes an Weizen (Puccinia recondita) oder gegen den Erreger der Braunspelzigkeit des Weizens (Leptosphaeria nodorum) oder gegen den Erreger der Netzflekkenkrankheit der Gerste (Pyrenophora teres) oder gegen den Erreger der Braunfleckenkrankheit an Gerste und Weizen (Cochliobolus sativus) oder zur Bekämpfung von Reiskrankheiten, wie beispielsweise gegen den Erreger der Reisfleckenkrankheit (Pyricularia oryzae) oder zur Bekämpfung von Krankheiten im Obst- und Gemüseanbau, wie beispielsweise gegen den Erreger des Bohnengrauschimmels (Botrytis cinerea) oder gegen den Erreger des Apfelschorfes (Venturia inaequalis) einsetzen.

Darüberhinaus zeigen die erfindungsgemäßen Wirkstoffe eine gute fungizide in vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

## Herstellungsbeispiele

### Beispiel 1

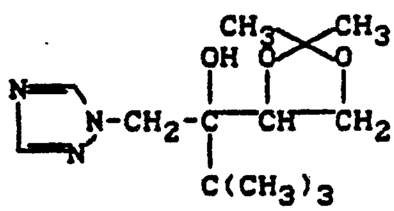

12,4 g (0,062 Mol) 2-t-Butyl-2-(2,2-dimethyl-1,3-dioxolan-4-yl)-oxiran, 5,1 g (0,074 Mol) Triazol und 3 g (0,074 Mol) Natriumhydroxid werden in 60 ml Dimethylformamid gelöst und 10 Stunden bei 100 °C gerührt. Zur Aufarbeitung wird die erkaltete Reaktionsmischung in Wasser gegeben, mit Essigester extrahiert, die organische Phase mit Wasser gewaschen, über Natriumsulfat getrocknet, im Vakuum eingeengt und der Rückstand durch Säulenchromatographie an Kieselgel (Laufmittel: Essigester/Cyclohexan 3:1) gereinigt.

Man erhält 4,4 g (26 % der Theorie) an 2,2-Dimethyl-1-(2,2-dimethyl-1,3-dioxolan-4-yl)-1-(1,2,4-triazol-1-yl-methyl)-propan-1-ol als Öl vom Brechungsindex $n_D^{20}$ 1.4890;
$^1$H-NMR ($CDCl_3$/Tetramethylsilan):
$\delta$ = 1.00 (s, 9H);
1.30 (s, 3H);
1.31 (s, 3H) ppm.

### Herstellung der Ausgangverbindungen

### Beispiel II-1

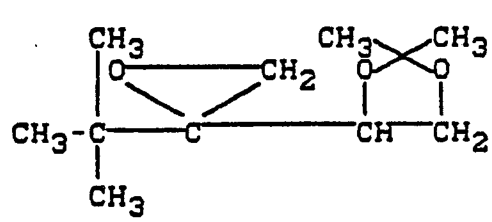

Zu 17,6 g (0.08 Mol) Trimethyloxosulfoniumiodid in 17,5 g (0,22 Mol) Dimethylsulfoxid gibt man bei Raumtemperatur 9 g (0,08 Mol) Kalium-t-butylat, rührt 6 Stunden bei Raumtemperatur, gibt dann tropfenweise unter Rühren 13 g (0,0698 Mol) 4-(2,2-Dimethylpropanoyl)-2,2-dimethyl-1,3-dioxolan zu und rührt weitere 8 Stunden bei Raumtemperatur und anschließend 1 Stunde bei Rückflußtemperatur. Zur Aufarbeitung wird die erkaltete Reaktionsmischung in Wasser gegeben, mit Dichlormethan extrahiert, die organische Phase mit Wasser gewaschen, über Natriumsulfat getrocknet und im Vakuum eingeengt.

Man erhält 12,4 g (89 % der Theorie) an 2-t-Butyl-2-(2,2-dimethyl-1,3-dioxolan-4-yl)-oxiran vom Bre-

chungsindex $n_D^{20}$ 1,4519.

Beispiel VII-1

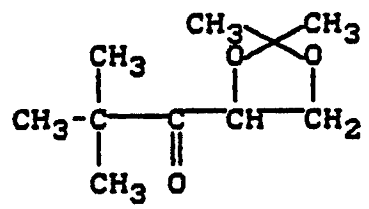

Zu 5,8 g (0,1 Mol) Aceton und 12,8 g 2-(2,2-Dimethylpropanoyl)-oxiran in 50 ml Tetrachlorkohlenstoff gibt man bei - 35 °C tropfenweise unter Rühren 5,2 g (0,02 Mol) Zinntetrachlorid, rührt nach beendeter Zugabe weitere 3 Stunden bei - 35 °C, läßt die Reaktionsmischung auf 0 °C kommen und gibt unter Eiskühlung 50 ml Wasser zu. Die organische Phase wird abgetrennt, mit Wasser gewaschen, über Natriumsulfat getrocknet, im Vakuum eingeengt und der Rückstand destilliert.

Man erhält 8,6 g (46 % der Theorie) an 4-(2,2-Dimethylpropanoyl)-2,2-dimethyl-1,3-dioxolan, MS (m/e = 186, 171, 141, 129, 101, 57).

Beispiel V-1

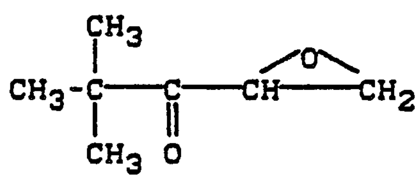

Zu 67,3 g (0,5 Mol) t-Butyl-chlormethyl-keton (vgl. z.B. Bull. Soc. Chim. Fr. 1970, 3641-3646) und 42,9 g (0,5 Mol) 35prozentiger wässriger Formaldehydlösung in 400 ml Methanol gibt man bei Raumtemperatur tropfenweise unter Rühren 44,4 g (0,5 Mol) einer 45prozentigen Natronlauge und erhitzt nach beendeter Zugabe 2 Stunden auf Rückflußtemperatur, wobei durch Zugabe von weiterer Natronlauge der pH-Wert auf pH 14 konstant gehalten wird. Zur Aufarbeitung wird mit wässriger Salzsäure neutralisiert, das Methanol im Vakuum abdestilliert, der Rückstand mit Dichlormethan extrahiert, über Natriumsulfat getrocknet und destilliert.

Man erhält 31,5 g (49 % der Theorie) an 2-(2,2-Dimethylpropanoyl)-oxiran vom Siedepunkt 72-75 °C bei 20 mbar.

$^1$H-NMR ($CDCl_3$)/Tetramethylsilan):

$\delta$ = 1,27 (s, 9H);

3,86 (m,1H) ppm.

In entsprechender Weise und gemäß den allgemeinen Angaben zur Herstellung erhält man die folgenden substituierten Dioxolane der allgemeinen Formel (I):

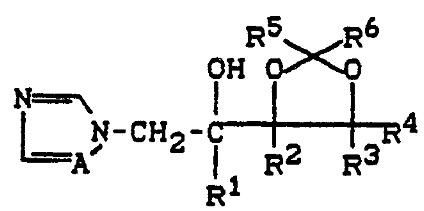

(I)

| Bsp. Nr. | $R^1$ | 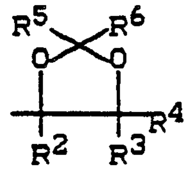 | A | physikalische Eigenschaften |
|---|---|---|---|---|
| 2 | $(CH_3)_3C$- | 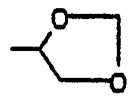 | N | $^1$H-NMR*): 1,02; 7,98; 8,15 |
| 3 | 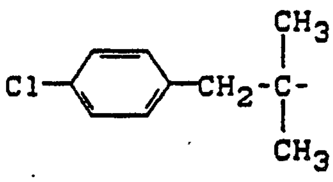 | 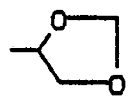 | N | Fp 65 °C |
| 4 | 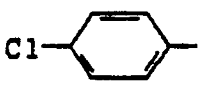 | 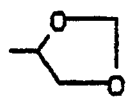 | N | Fp 175 °C |
| 5 | 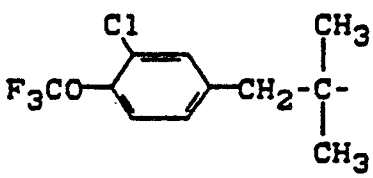 | 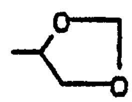 | N | $^1$H-NMR*): 8,02; 8,21 |

| Bsp. Nr. | $R^1$ | 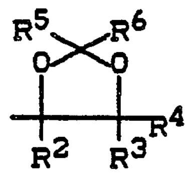 | A | physikalische Eigenschaften |
|---|---|---|---|---|
| 6 | 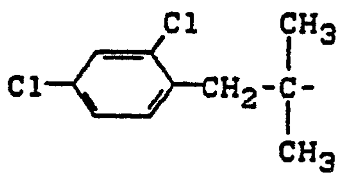 | 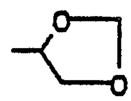 | N | $^1H$-NMR*): 4,77 |
| 7 | 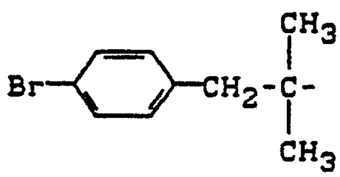 | 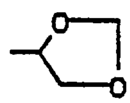 | N | $^1H$-NMR*): 0,89; 0,895 |
| 8 | 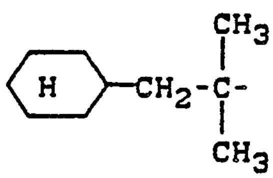 | 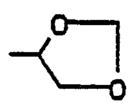 | N | $^1H$-NMR*): 4,72; 4,91; 1,00 (Diastereomeres A) |
| 9 | 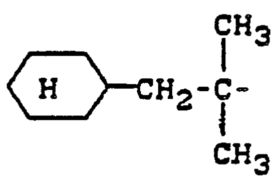 | 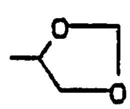 | N | $^1H$-NMR*): 0,9 (Diastereomeres B) |
| 10 | 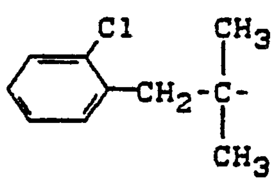 | 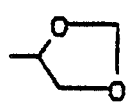 | N | $^1H$-NMR*): 0,95, 0,99 |
| 11 | 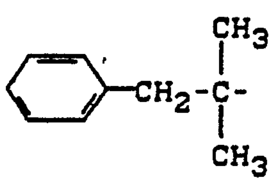 | 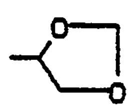 | N | $n_D^{20}$ 1.5346 |

| Bsp. Nr. | $R^1$ | 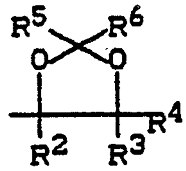 | A | physikalische Eigenschaften |
|---|---|---|---|---|
| 12 | 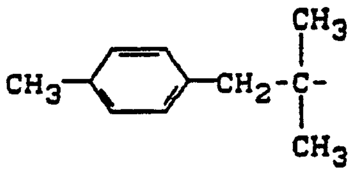 | 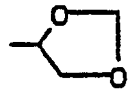 | N | Fp 102-104° C |
| 13 | 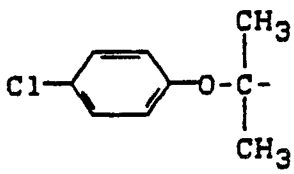 | 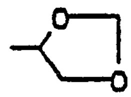 | N | $^1H$-NMR*): 1,26; 1,33 |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurden die nachstehend aufgeführten Verbindungen als Vergleichssubstanzen eingesetzt:

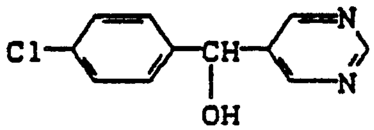

(A)

4-Chlorphenyl-5-pyrimidiyl-methanol

(bekannt aus US 4 417 050)

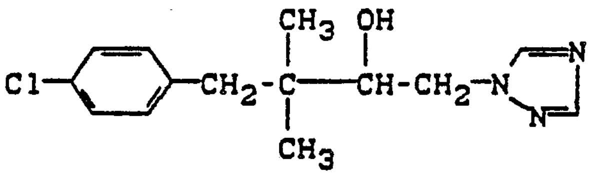

(B)

4-(4-Chlorphenyl)-3,3-dimethyl-1-(1,2,4-triazol-1-yl)-2-butanol

(bekannt aus EP 55 833).

Beispiel A

Venturia-Test (Apfel) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Gegenüber der Verbindung B zeigen die Verbindungen der Herstellungsbeispiele 3 und 4 bei einer beispielhaften Konzentration von 10 ppm einen sehr hohen Wirkungsgrad. Die Verbindung B ist nahezu unwirksam.

Beispiel B

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Antrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Gegenüber der Verbindung A zeigt die Verbindungen des Herstellungsbeispiels 3 bei einer beispielhaften Konzentration von 0,025 % Wirkstoffzubereitung einen sehr hohen Wirkungsgrad. Die Verbindung A ist nahezu unwirksam.

Beispiel C

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen vonErysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Gegenüber der Verbindung (B) zeigen die Verbindungen der Herstellungsbeispiele (3), (5), (10) und (12) bei einer beispielhaften Konzentration von 0,0025 Gew.-% einen um 50 bis 75 % höheren Wirkungsgrad.

Beispiel D

Cochliobolus sativus-Test (Gerste)/protektiv

Lösungsmittel: 100 Gewichtsteile Dimethylformamid
Emulgator :0,25 Gewichtsteile Alkylarylpolyglykol ether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Gegenüber der Verbindung (B) zeigen die Verbindungen der Herstellungsbeispiele (3), (5), (10) und (12) bei einer beispielhaften Konzentration von 0,025 Gew.-% einen um 63 bis 75 % höheren Wirkungsgrad.

Beispiel E

Pyrenophora teres-Test (Gerste) / protektiv

Lösungsmittel: 100 Gewichtsteile Dimethylformamid
Emulgator: 0,25 Gewichtsteile Alkylarylpolyglkyolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca 20 °C und einer relativen Luftfeuchtigkeit von ca. 80 % aufgestellt.

7 Tage nach der Inokulation erfolgt die Auswertung.

Gegenüber der Verbindung (B) zeigen die Verbindungen der Herstellungsbeispiele (3), (5), (10) und (12) bei einer beispielhaften Konzentration von 0,025 Gew.-% einen um 46 bis 55 % höheren Wirkungsgrad.

## Ansprüche

1. Substituierte Dioxolane der Formel

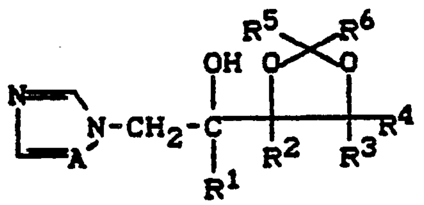

(I),

in welcher
$R^1$ für gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkylalkyl, gegebenenfalls substituiertes Aryl, gegebenenfalls substituiertes Aryloxy, gegebenenfalls substituiertes Arylthio, gegebenenfalls substituiertes Arylsulfinyl, gegebenenfalls substituiertes Arylsulfonyl, gegebenenfalls substituiertes Arylalkyl, gegebenenfalls substituiertes Aryloxyalkyl, gegebenenfalls substituiertes Arylthioalkyl, gegebenenfalls substituiertes Arylsulfinylalkyl, gegebenenfalls substituiertes Arylsulfonylalkyl, gegebenenfalls substituiertes Aralkyloxyalkyl, gegebenenfalls substituiertes Aralkylthioalkyl, gegebenenfalls substituiertes Aralkylsulfinylalkyl oder gegebenenfalls substituiertes Aralkylsulfonylalkyl steht,
$R^2$, $R^3$, $R^4$, $R^5$ und $R^6$ unabhängig voneinander für Wasserstoff, Alkyl oder Cycloalkyl stehen, oder
$R^3$ und $R^4$ gemeinsam für zweifach verknüpftes Alkandiyl stehen und/oder
$R^5$ und $R^6$ gemeinsam für zweifach verknüpftes Alkandiyl stehen und
A für Stickstoff oder eine CH-Gruppe steht;
sowie deren Säureadditions-Salze und Metallsalz-Komplexe.

2. Substituierte Dioxolane gemäß Anspruch 1, wobei in der Formel (I)
$R^1$ für geradkettiges oder verzweigtes Alkyl mit 1 bis 12 Kohlenstoffatomen, geradkettiges oder verzweigtes Alkenyl mit 2 bis 12 Kohlenstoffatomen, geradkettiges oder verzweigtes Alkinyl mit 2 bis 12 Kohlenstoffatomen, geradkettiges oder verzweigtes
Alkoxyalkyl
mit 1 bis 8 Kohlenstoffatomen im Alkoxyteil und 1 bis 8 Kohlenstoffatomen im Alkylteil, geradkettiges oder verzweigtes Alkylthioalkyl mit 1 bis 8 Kohlenstoffatomen im Alkylthioteil und 1 bis 8 Kohlenstoffatomen im Alkylteil, geradkettiges oder verzweigtes Alkylsulfinylalkyl mit 1 bis 8 Kohlenstoffatomen im Alkyl sulfinylteil und 1 bis 8 Kohlenstoffatomen im Alkylteil, geradkettiges oder verzweigtes Alkylsulfonylalkyl 1 bis 8 Kohlenstoffatomen im Alkylsulfonylteil und 1 bis 8 Kohlenstoffatomen im Alkylteil, geradkettiges oder verzweigtes Alkoximinoalkyl mit 1 bis 8 Kohlenstoffatomen im Alkoxyteil und 1 bis 8 Kohlenstoffatomen im

Alkylteil, geradkettiges oder verzweigtes Hydroximinoalkyl mit 1 bis 8 Kohlenstoffatomen, geradkettiges oder verzweigtes Cyanalkyl mit 1 bis 8 Kohlenstoffatomen im Alkylteil, geradkettiges oder verzweigtes Halogenalkyl mit 1 bis 8 Kohlenstoffatomen und 1 bis 9 gleichen oder verschiedenen Halogenatomen, geradkettiges oder verzweigtes Halogenalkoxyalkyl mit 1 bis 8 Kohlenstoffatomen im Alkoxyteil und 1 bis 8 Kohlenstoffatomen im Alkylteil und mit 1 bis 9 gleichen oder verschiedenen Halogenatomen, geradkettiges oder verzweigtes Halogenalkylthioalkyl mit 1 bis 8 Kohlenstoffatomen im Alkylthioteil und 1 bis 8 Kohlenstoffatomen im Alkylteil und mit 1 bis 9 gleichen oder verschiedenen Halogenatomen, geradkettiges oder verzweigtes Halogenalkylsulfinylalkyl mit 1 bis 8 Kohlenstoffatomen im Halogenalkylteil und 1 bis 8 Kohlenstoffatomen im Alkylteil und 1 bis 9 gleichen oder verschiedenen Halogenatomen, geradkettiges oder verzweigtes Halogenalkylsulfonylalkyl mit 1 bis 8 Kohlenstoffatomen im Halogenalkylteil und 1 bis 8 Kohlenstoffatomen im Alkylteil und 1 bis 9 gleichen oder verschiedenen Halogenatomen, geradkettiges oder verzweigtes für Halogenalkenyl mit 2 bis 12 Kohlenstoffatomen und 1 bis 9 gleichen oder verschiedenen Halogenatomen, geradkettiges oder verzweigtes Halogenalkinyl mit 2 bis 12 Kohlenstoffatomen und 1 bis 9 gleichen oder verschiedenen Halogenatomen, für Dioxolanylalkyl mit 1 bis 6 Kohlenstoffatomen im geradkettigen oder verzweigten Alkylteil, Dithiolanylalkyl mit 1 bis 6 Kohlenstoffatomen im geradkettigen oder verzweigten Alkylteil, Dioxanylalkyl mit 1 bis 6 Kohlenstoffatomen im geradkettigen oder verzweigten Alkylteil oder für Dithianylalkyl mit 1 bis 6 Kohlenstoffatomen im geradkettigen oder verzweigten Alkylteil steht,

oder

$R^1$ für Cycloalkyl mit 3 bis 7 Kohlenstoffatomen steht, wobei jeder dieser Cycloalkylreste einfach oder mehrfach, gleichartig oder verschieden substituiert sein kann durch Halogen und/oder Alkyl mit 1 bis 4 Kohlenstoffatomen,

oder

$R^1$ für Cycloalkyl mit 3 bis 7 Kohlenstoffatomen in Cycloalkylteil und 1 bis 8 Kohlenstoffatomen im geradkettigen oder verzweigten Alkylteil steht, wobei jeder dieser Reste im Cycloalkylteil einfach oder mehrfach, gleichartig oder verschieden substituiert sein kann durch Halogen und/oder Alkyl mit 1 bis 4 Kohlenstoffatomen,

oder

$R^1$ für Aryl mit 6 bis 10 Kohlenstoffatomen, Aryloxy mit 6 bis 10 Kohlenstoffatomen, Arylthio mit 6 bis 10 Kohlenstoffatomen, Arylsulfinyl mit 6 bis 10 Kohlenstoffatomen, Arylsulfonyl mit 6 bis 10 Kohlenstoffatomen, Arylalkyl mit 6 bis 10 Kohlenstoffatomen im Arylteil und 1 bis 8 Kohlenstoffatomen in geradkettigen oder verzweigten Alkylteil, Aryloxyalkyl mit 6 bis 10 Kohlenstoffatomen im Arylteil und 1 bis 8 Kohlenstoffatomen im geradkettigen oder verzweigten Alkylteil, Arylthioalkyl mit 6 bis 10 Kohlenstoffatomen im Arylteil und 1 bis 8 Kohlenstoffatomen im geradkettigen oder verzweigten Alkylteil, Arylsulfinylalkyl mit 6 bis 10 Kohlenstoffatomen im Arylteil und 1 bis 8 Kohlenstoffatomen im geradkettigen oder verzweigten Alkylteil, Arylsulfonylalkyl mit 6 bis 10 Kohlenstoffatomen im Arylteil und 1 bis 8 Kohlenstoffatomen im geradkettigen oder verzweigten Alkylteil, Aralkyloxyalkyl mit 6 bis 10 Kohlenstoffatomen im Arylteil und 1 bis 8 Kohlenstoffatomen in jedem geradkettigen oder verzweigten Alkylteil, Aralkylthioalkyl mit 6 bis 10 Kohlenstoffatomen im Arylteil und 1 bis 8 Kohlenstoffatomen in jedem geradkettigen oder verzweigten Alkylteil, Aralkylsulfinylalkyl mit 6 bis 10 Kohlenstoffatomen im Arylteil und 1 bis 8 Kohlenstoffatomen in jedem geradkettigen oder verzweigten Alkylteil oder für Aralkylsulfonylalkyl mit 6 bis 10 Kohlenstoffatomen im Arylteil und 1 bis 8 Kohlenstoffatomen in jedem geradkettigen oder ver zweigten Alkylteil steht, wobei jeder der zuvor genannten Aryl-Reste im Arylteil einfach oder mehrfach, gleichartig oder verschieden substituiert sein kann durch Halogen, Cyano, Nitro, geradkettiges oder verzweigtes Alkyl, geradkettiges oder verzweigtes Alkoxy, geradkettiges oder verzweigtes Alkylthio, geradkettiges oder verzweigtes Alkylsulfinyl oder geradkettiges oder verzweigtes Alkylsulfonyl mit jeweils 1 bis 4 Kohlenstoffatomen, geradkettiges oder verzweigtes Halogenalkyl, geradkettiges oder verzweigtes Halogenalkoxy, geradkettiges oder verzweigtes Halogenalkylthio, geradkettiges oder verzweigtes Halogenalkylsulfinyl oder geradkettiges oder verzweigtes Halogenalkylsulfonyl mit jeweils 1 bis 4 Kohlenstoffatomen und 1 bis 9 gleichen oder verschiedenen Halogenatomen, geradkettiges oder verzweigtes Alkoxycarbonyl oder geradkettiges oder verzweigtes Alkoximinoalkyl mit jeweils 1 bis 4 Kohlenstoffatomen in den einzelnen Alkylteilen, unsubstituiertes oder einfach oder mehrfach, gleich oder verschieden durch Halogen substituiertes, zweifach verknüpftes Dioxyalkylen, Cycloalkyl mit 3 bis 7 Kohlenstoffatomen und unsubstituiertes oder einfach oder mehrfach, gleich oder verschieden durch Halogen und/oder geradkettiges oder verzweigtes Alkyl mit 1 bis 4 Kohlenstoffatomen substituiertes Phenyl oder Phenoxy,

$R^2$, $R^3$, $R^4$, $R^5$ und $R^6$ unabhängig voneinander jeweils für Wasserstoff, für geradkettiges oder verzweigtes Alkyl mit 1 bis 6 Kohlenstoffatomen oder für Cycloalkyl mit 3 bis 7 Kohlenstoffatomen stehen, wobei auch entweder $R^3$ und $R^4$ gemeinsam oder $R^5$ und $R^6$ gemeinsam für einen jeweils zweifach verknüpften

Alkandiylrest mit 2 bis 6 Kohlenstoffatomen stehen können und
A für Stickstoff oder eine CH-Gruppe steht.

3. Verfahren zur Herstellung von substituierten Dioxolanen der allgemeinen Formel (I),

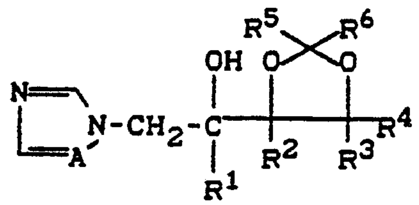

(I)

in welcher
$R^1$ für gegebenenfalls substituiertes Alkyl, gegebenenfalls substituiertes Alkenyl, gegebenenfalls substituiertes Alkinyl, gegebenenfalls substituiertes Cycloalkyl oder gegebenenfalls substituiertes Aryl, gegebenenfalls substituiertes Aryloxy, gegebenenfalls substituiertes Arylthio, gegebenenfalls substituiertes Arylsulfinyl, gegebenenfalls substituiertes Arylsulfonyl, gegebenenfalls substituiertes Arylalkyl, gegebenenfalls substituiertes Aryloxyalkyl, gegebenenfalls substituiertes Arylthioalkyl, gegebenenfalls substituier tes Arylsulfinylalkyl, gegebenenfalls substituiertes Arylsulfonylalkyl, gegebenenfalls substituiertes Aralkyloxyalkyl, gegebenenfalls substituiertes Aralkylthioalkyl, gegebenenfalls substituiertes Aralkylsulfinylalkyl oder gegebenenfalls substituiertes Aralkylsulfonylalkyl steht,
$R^2$, $R^3$, $R^4$, $R^5$ und $R^6$ unabhängig voneinander jeweils für Wasserstoff, Alkyl oder Cycloalkyl stehen, wobei auch entweder $R^3$ und $R^4$ gemeinsam oder $R^5$ und $R^6$ gemeinsam für einen zweifach verknüpften Alkandiylrest stehen können und
A für Stickstoff oder eine CH-Gruppe steht,
sowie deren Säureadditionssalze und Metallsalzkomlexe, dadurch gekennzeichnet, daß man Oxiranyldioxolane der Formel (II),

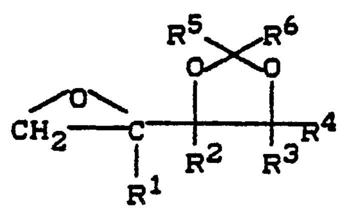

(II)

in welcher,
$R^1$, $R^2$, $R^3$, $R^4$, $R^5$ und $R^6$ die oben angegebene Bedeutung haben,
mit Azolen der Formel (III),

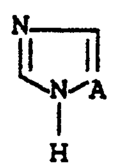

(III)

in welcher
A die oben angegebene Bedeutung hat,
gegebenenfalls in Gegenwart eines Verdünnungsmittels und gegebenenfalls in Gegenwart eines Reaktionshilfsmittels umsetzt und gegebenenfalls anschließend eine Säure oder ein Metallsalz addiert.

4. Mikrobizide Mittel, gekennzeichnet durch einen Gehalt an mindestens einer Verbindung der allgemeinen Formel (I) gemäß den Anspruch 1.

5. Verwendung von Verbindungen der allgemeinen Formel (I) gemäß den Anspruch 1 zur Bekämpfung von Mikroorganismen.

6. Oxiranyldioxolane der Formel (IIa),

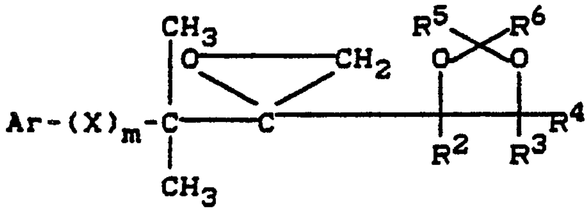

(IIa)

in welcher
Ar für gegebenenfalls substituiertes Aryl steht,
X für Sauerstoff, Schwefel, Sulfinyl, Sulfonyl oder für eine der Gruppen, $-CH_2-$, $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$ steht,
m für eine Zahl 0 oder 1 steht,
n jeweils für eine Zahl 0, 1 oder 2 steht und
$R^2$, $R^3$, $R^4$, $R^5$ und $R^6$ unabhängig voneinander für Wasserstoff, Alkyl oder Cycloalkyl stehen, oder
$R^3$ und $R^4$ gemeinsam für zweifach verknüpftes Alkandiyl stehen und/oder
$R^5$ und $R^6$ gemeinsam für zweifach verknüpftes Alkandiyl stehen.

7. Verfahren zur Herstellung von Oxiranyldioxolanen der Formel (IIa) gemäß Anspruch 6, dadurch gekennzeichnet, daß man Vinylketone der Formel (IVa),

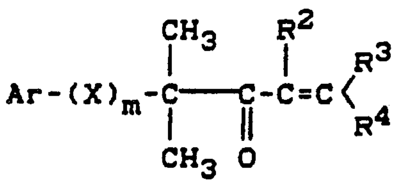

(IVa)

in welcher
Ar, X, $R^2$, $R^3$, $R^4$ und m die in Anspruch 6 angegebene Bedeutung haben,
mit einem Oxidationsmittel gegebenenfalls in Gegenwart eines Verdünnungsmittels bei Temperaturen zwischen 10 °C und 60 °C epoxidiert und dann die so erhältlichen Oxiranylketone der Formel (Va),

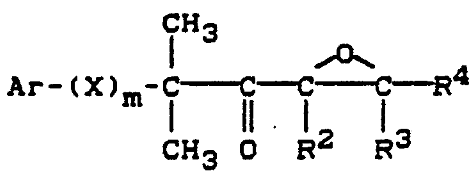

(Va)

in welcher
Ar, X, $R^2$, $R^3$, $R^4$ und m die in Anspruch 6 angegebenen Bedeutung haben,
in einer 2. Stufe mit Aldehyden oder Ketonen der Formel (VI),

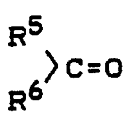

(VI)

in welcher
$R^5$ und $R^6$ die in Anspruch 6 angegebene Bedeutung haben,
gegebenenfalls in Gegenwart eines Katalysators und gegebenenfalls in Gegenwart eines Verdünnungsmittels bei Temperaturen zwischen - 80 °C und + 50 °C umsetzt und dann die so erhältlichen Dioxolanylketone der Formel (VIIa),

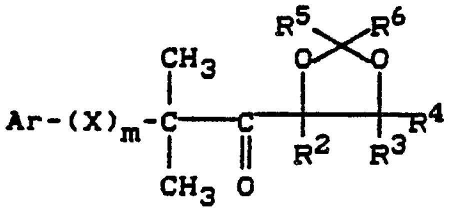

(VIIa)

in welcher
Ar, X, $R^2$, $R^3$, $R^4$, $R^5$, $R^6$ und m die in Anspruch 6 angegebene Bedeutung haben, entweder mit Dimethyloxosulfoniummethylid der Formel (VIII)

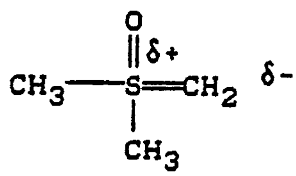

(VIII)

in Gegenwart eines Verdünnungsmittels bei Temperaturen zwischen 20 °C und 80 °C umsetzt oder mit Trimethylsulfoniummethylsulfat der Formel (IX)

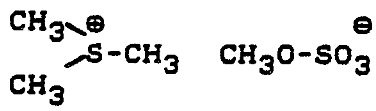

(IX)

in Gegenwart eines Verdünnungsmittels und in Gegenwart einer Base bei Temperaturen zwischen 0 °C und 60 °C umsetzt.

8. Dioxolanylketone der Formel (VIIa),

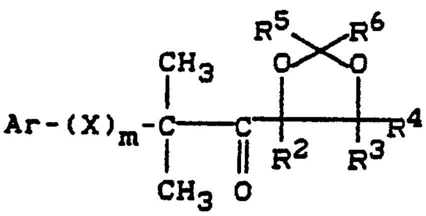

(VIIa)

in welcher
Ar für gegebenenfalls substituiertes Aryl steht,
X für Sauerstoff, Schwefel, Sulfinyl, Sulfonyl oder für eine der Gruppen, $-CH_2-$, $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$ steht,
m für eine Zahl 0 oder 1 steht,
n jeweils für eine Zahl 0, 1 oder 2 steht und
$R^2$, $R^3$, $R^4$, $R^5$ und $R^6$ unabhängig voneinander für Wasserstoff, Alkyl oder Cycloalkyl stehen, wobei auch entweder $R^3$ und $R^4$ gemeinsam oder $R^5$ und $R^6$ gemeinsam für einen zweifach verknüpften Alkandiylrest stehen können.

9. Verfahren zur Herstellung der Dioxolanylketone der Formel (VIIa) gemäß Anspruch 8, dadurch gekennzeichnet, daß man Vinylketone der Formel

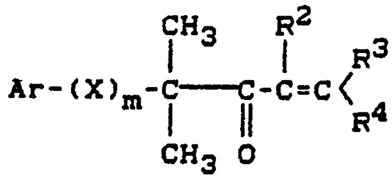

(IVa),

in welcher
Ar, X, $R^2$, $R^3$, $R^4$ und m die in Anspruch 13 angegebene Bedeutung haben,
mit einem Oxidationsmittel, gegebenenfalls in Gegenwart eines Verdünnungsmittels, bei Temperaturen zwischen 10 °C und 60 °C epoxidiert und dann die so erhältlichen Oxiranylketone der Formel (Va),

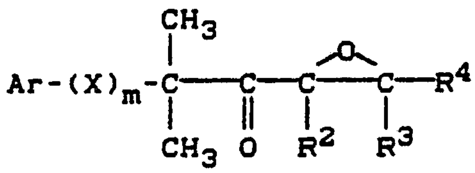

(Va)

in welcher
Ar, X, $R^2$, $R^3$, $R^4$ und m die in Anspruch 13 angegebene Bedeutung haben,
in einer 2. Stufe mit Aldehyden oder Ketonen der Formel (VI),

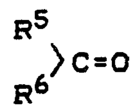

(VI)

in welcher
$R^5$ und $R^6$ die in Anspruch 8 angegebene Bedeutung haben,
gegebenenfalls in Gegenwart eines Katalysators und gegebenenfalls in Gegenwart eines Verdünnungsmittels bei Temperatur zwischen - 80 °C und + 50 °C umsetzt.

10. Oxiranylketone der Formel (Va),

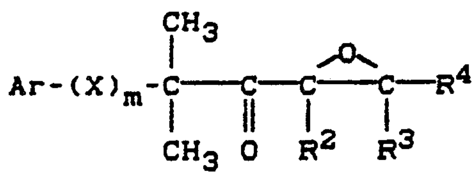

(Va)

in welcher
Ar für gegebenenfalls substituiertes Aryl steht,
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$ steht,
m für eine Zahl 0 oder 1 steht,
n jeweils für eine Zahl 0, 1 oder 2 steht und
$R^2$, $R^3$, $R^4$, $R^5$ und $R^6$ die oben angegebene Bedeutung haben.

11. Verfahren zur Herstellung von Oxiranylketonen der Formel (Va) gemäß Anspruch 10, dadurch gekennzeichnet, daß man Vinylketone der Formel (IVa)

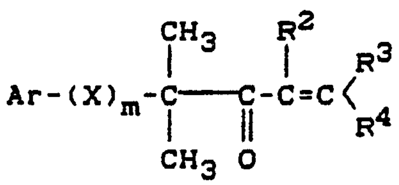

(IVa),

in welcher
Ar, X, $R^2$, $R^3$, $R^4$ und m die in Anspruch 10 angegebenen Bedeutungen haben,
mit einem Oxidationsmittel, gegebenenfalls in Gegenwart eines Verdünnungsmittels, bei Temperaturen zwischen 10 °C und 60 °C epoxidiert.

12. α-Halogenketone der Formel (XIIa)

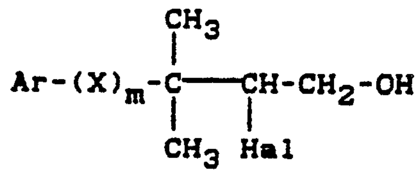
(XIIa)

in welcher
Hal für Halogen steht
Ar für gegebenenfalls substituiertes Aryl steht,
X für Sauerstoff, Schwefel, Sulfinyl, Sulfonyl oder für eine der Gruppen $-CH_2-$, $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$ steht,
m für eine Zahl 0 oder 1 steht und
n jeweils für eine Zahl 0, 1 oder 2 steht.

13. Verfahren zur Herstellung von α-Halogenketonen der Formel (XIIa) gemäß Anspruch 12, dadurch gekennzeichnet, daß man Halogenmethylketone der Formel (XIa),

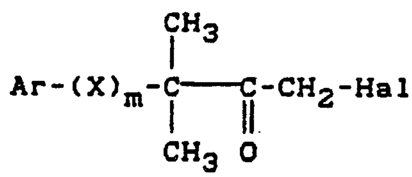
(XIa)

in welcher
Ar, X, Hal und m die in Anspruch 12 angegebene Bedeutung haben,
mit Formaldehyd gegebenenfalls in Gegenwart eines Verdünnungsmittels, und gegebenenfalls in Gegenwart eines Reaktionshilfsmittels bei Temperaturen zwischen 0° C und 60° C umsetzt.

Europäisches Patentamt

# EUROPÄISCHER TEILRECHERCHENBERICHT,

der nach Regel 45 des Europäischen Patentübereinkommens für das weitere Verfahren als europäischer Recherchenbericht gilt

Nummer der Anmeldung

EP 89113967.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 5) |
|---|---|---|---|
| A | DE - B - 1 241 831 (GYÖGYSZERIPARI) * Anspruch 1 * -- | 1 | C 07 D 405/06<br>C 07 D 407/04<br>C 07 D 317/26<br>C 07 D 303/32<br>C 07 C 33/46<br>C 07 C 43/178<br>C 07 C 43/23<br>C 07 C 317/24<br>C 07 C 323/22<br>A 01 N 43/50<br>A 01 N 43/647 |
| A | CHEMICAL ABSTRACTS, Band 87, Nr. 1, 4. Juli 1977, Columbus, Ohio, USA ISHIDO, YOSHIHARU et al. "Synthetic studies by the use of carbonates. Part IX. Reactions of D-mannitol carbonate derivatives catalyzed by tetraethylammonium bromide" Seite 513, Spalte 1, Zusammenfassung-Nr. 6 280j & Carbohydr. Res. 1976, 52(1), 49-61 -- | 1 | |
| A | CHEMICAL ABSTRACTS, Band 70, Nr. 13, 31. März 1969, Columbus, Ohio, USA TANAKA, TERUO et al. "O-Isopropylidene derivatives | 1 | |

RECHERCHIERTE SACHGEBIETE (Int. Cl. 5)

C 07 D 405/00
C 07 D 407/00
C 07 D 317/00
C 07 D 303/00
C 07 C 33/00
C 07 C 43/00
C 07 C 317/00
C 07 C 323/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-4,6-13
Unvollständig recherchierte Patentansprüche: 5
Nicht recherchierte Patentansprüche: -
Grund für die Beschränkung der Recherche:

(Art. 52(4) EPÜ) Verfahren zur therapeutischen Behandlung des menschlichen und tierischen Körpers)

| Recherchenort | Abschlußdatum der Recherche | Prüfer |
|---|---|---|
| WIEN | 17-10-1989 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 03.82

Europäisches Patentamt

# EUROPÄISCHER TEILRECHERCHENBERICHT

Nummer der Anmeldung
-2-
EP 89113967.7

| EINSCHLÄGIGE DOKUMENTE | | | KLASSIFIKATION DER ANMELDUNG (Int. Cl.4) |
|---|---|---|---|
| Kategorie | Kennzeichnung des Dokuments mit Angabe, soweit erforderlich, der maßgeblichen Teile | betrifft Anspruch | |
| | of riboflavine"<br>Seite 418, Spalte 1, Zusammenfassung-Nr. 58 196e<br>& Yakugaku Zasshi 1968, 88(10), 1313-17<br>-- | | |
| A | CHEMICAL ABSTRACTS, Band 96, Nr. 17, 26. April 1982, Columbus, Ohio, USA<br>SEEBACH, DIETER et al. "Enantioselective generation and diastereoselective reactions of chiral enolates derived from alpha-hetero-substituted carboxylic acids. Preliminary communication"<br>Seite 669, Spalte 2, Zusammenfassung-Nr. 141 929d<br>& Helv. Chim. Acta 1981, 64(8), 2704-8<br>-- | 6,8 | RECHERCHIERTE SACHGEBIETE (Int. Cl.4) |
| A | CHEMICAL ABSTRACTS, Band 102, Nr. 9, 4. März 1985, Columbus, Ohio, USA<br>HEATHCOCK, CLAYTON H. et al. "Acyclic stereoselection. 23. Lactaldehyde enolate equivalents"<br>Seite 523, Spalte 2, Zusammenfassung-Nr. 77 968d<br>& J. Am. Chem. Soc. 1984, 106(26),8161-74<br>-- | 8 | |
| A | CHEMICAL ABSTRACTS, Band 106, Nr. 21, 25. Mai 1987, Columbus, Ohio, USA<br>SATO, FUMIE "Chiral glycerol derivatives"<br>Seite 771, Spalte 2, Zusammenfassung-Nr. 176 799p<br>& Jpn. Kokai Tokkyo Koho JP 61,205,274<br>-- | 8 | |
| A | CHEMICAL ABSTRACTS, Band 109, Nr. 5, 1. August 1988, | 8 | |

Europäisches Patentamt

**EUROPÄISCHER TEILRECHERCHENBERICHT**

Nummer der Anmeldung
-3-
EP 89113967.7

| EINSCHLÄGIGE DOKUMENTE | | | KLASSIFIKATION DER ANMELDUNG (Int. Cl.4) |
|---|---|---|---|
| Kategorie | Kennzeichnung des Dokuments mit Angabe, soweit erforderlich, der maßgeblichen Teile | betrifft Anspruch | |
| | Columbus, Ohio, USA<br>YUYA, MASAKAZU et al.<br>"Preparation of plant-growth hormone brassinosteroid intermediates"<br>Seite 641, Spalte 1, Zusammenfassung-Nr. 38 060b<br>& Jpn. Kokai Tokkyo Koho JP 62,201,897<br>-- | | |
| A | CHEMICAL ABSTRACTS, Band 101, Nr. 21, 19. November 1984, Columbus, Ohio, USA<br>SEEBACH, DIETER et al.<br>"Alpha-Alkylation of alpha--heterosubstituted carboxylic acids without racemization. EPC-syntheses of tertiary alcohols and thiols"<br>Seite 755, Spalte 2, Zusammenfassung-Nr. 191 762m<br>& Tetrahedron 1984, 40(8), 1313-24<br>-- | 8 | RECHERCHIERTE SACHGEBIETE (Int. Cl.4) |
| A | CHEMICAL ABSTRACTS, Band 103, Nr. 13, 30. September 1985, Columbus, Ohio, USA<br>IRIE, HIROSHI et al. "An approach to a synthesis of the host-specific toxins AK-toxin I and II, starting from vitamin C as a chiral material"<br>Seite 602, Spalte 2, Zusammenfassung-Nr. 104 770n<br>& Chem. Pharm. Bull. 1985, 33(3), 1313-15<br>-- | 10 | |
| A | DE - B - 1 230 416<br>(HOOKER)<br>* Anspruch 1 *<br>-- | 12 | |
| A | EP - B1 - 0 046 532<br>(BAYER)<br>* Anspruch 1 *<br>---- | 12 | |